# EUROPEAN PATENT APPLICATION

(11) **EP 1 795 896 A2**
(43) Date of publication of application: **13.06.2007**
(21) Application number: 06077074.0
(22) Date of filing: 22.11.2006
(51) Int. Cl.: G01N 33/50

(54) **Method for the identification and relative quantification of proteins based on the selective isolation of RRNK peptides for the simplification of complex mixtures of proteins**

(30) Priority: 22.11.2005 CU 230005
(71) Applicant: CENTRO DE INGENIERIA GENETICA Y BIOTECNOLOGIA (CIGB), Ciudad de la Habana 10600 (CU)
(72) Inventor: Puente, Aniel Sanchez, 11300, Ciudad de la Habana (CU); Gonzalez Lopez, Luis Javier, Mun. Playa 12100, Ciudad de la Habana (CU); Gil Valdés, Jeonvanis, Arroyo Naranjo 10900 Ciudad de la Habana (CU); Betancourt Nunez, Lazaro Hiram, 12500 Ciudad de la Habana (CU); Besada Pérez, Vladimir Armando, 19140, Ciudad de la Habana (CU); Fernandez de Cossio Dorta-Duque, Jorge, 19140, Ciudad de la Habana (CU); Alvarez Gil, Félix Modesto, 10600, Ciudad de la Habana (CU); Padron Palomares, Gabriel Ramon, 12100, Ciudad de la Habana (CU); Gomez, Yassel Ramos, 10600, La Habana (CU)
(74) Representative: van Loon, C.J.J.

(57) **Abstract**

A method based on the selective isolation of peptides originated by the cleavage at the C-terminal end of the arginine residues and do not possess lysine inside their sequences (péptidos RRnK), is described. The method is based on the blocking of amino groups of the LEP peptides and the separation of the RRnK peptides and the modified peptides by using a chromatography column or a chemical reaction of the modified peptides with a solid support. The method simplifies the complex mixtures of peptides by isolating selectively an average of 4 peptides/protein and it guarantees a coverage of 88% of the proteomes, its specificity and selectivity are very high (>95%), it is compatible with different types of isotopic labeling (¹³C, ¹⁸O or ¹⁵N) and it is useful for the determination of the differential expression of proteins without the necessity of using the two-dimensional electrophoresis.

SEQUENCE LIST

<110> Centro de Ingeniería Genética y Biotecnología

<120> Method for the identification and relative quantification of proteins based
on the selective isolation of RRnK peptides for the simplification of complex
mixtures of proteins.

<130> selective isolation of peptide RRnK

<140>
<141>

<160> 6

<170> PatentIn Ver. 2.1

<210> 1
<211> 415
<212> PRT
<213> Streptococcus equisimilis

<220>
<221> PEPTIDE
<222> (1)..(415)
<223> streptokinase [Streptococcus equisimilis]

<400> 1
Met Ile Ala Gly Pro Glu Trp Leu Leu Asp Arg Pro Ser Val Asn Asn
1               5                   10                  15

Ser Gln Leu Val Val Ser Val Ala Gly Thr Val Glu Gly Thr Asn Gln
            20                  25                  30

Asp Ile Ser Leu Lys Phe Phe Glu Ile Asp Leu Thr Ser Arg Pro Ala
        35                  40                  45

His Gly Gly Lys Thr Glu Gln Gly Leu Ser Pro Lys Ser Lys Pro Phe
    50                  55                  60

Ala Thr Asp Ser Gly Ala Met Pro His Lys Leu Glu Lys Ala Asp Leu
65                  70                  75                  80

Leu Lys Ala Ile Gln Glu Gln Leu Ile Ala Asn Val His Ser Asn Asp
                85                  90                  95

Asp Tyr Phe Glu Val Ile Asp Phe Ala Ser Asp Ala Thr Ile Thr Asp
            100                 105                 110

Arg Asn Gly Lys Val Tyr Phe Ala Asp Lys Asp Gly Ser Val Thr Leu
        115                 120                 125

Pro Thr Gln Pro Val Gln Glu Phe Leu Leu Ser Gly His Val Arg Val
    130                 135                 140

Arg Pro Tyr Lys Glu Lys Pro Ile Gln Asn Gln Ala Lys Ser Val Asp
145                 150                 155                 160

Val Glu Tyr Thr Val Gln Phe Thr Pro Leu Asn Pro Asp Asp Asp Phe
                165                 170                 175

Arg Pro Gly Leu Lys Asp Thr Lys Leu Leu Lys Thr Leu Ala Ile Gly
            180                 185                 190

Asp Thr Ile Thr Ser Gln Glu Leu Leu Ala Gln Ala Gln Ser Ile Leu
        195                 200                 205

Asn Lys Thr His Pro Gly Tyr Thr Ile Tyr Glu Arg Asp Ser Ser Ile
    210                 215                 220

Val Thr His Asp Asn Asp Ile Phe Arg Thr Ile Leu Pro Met Asp Gln
225                 230                 235                 240

Glu Phe Thr Tyr His Val Lys Asn Arg Glu Gln Ala Tyr Glu Ile Asn
                245                 250                 255

Lys Lys Ser Gly Leu Asn Glu Glu Ile Asn Asn Thr Asp Leu Ile Ser
            260                 265                 270

Glu Lys Tyr Tyr Val Leu Lys Lys Gly Glu Lys Pro Tyr Asp Pro Phe
        275                 280                 285

Asp Arg Ser His Leu Lys Leu Phe Thr Ile Lys Tyr Val Asp Val Asn
    290                 295                 300

Thr Asn Glu Leu Leu Lys Ser Glu Gln Leu Leu Thr Ala Ser Glu Arg
305                 310                 315                 320

Asn Leu Asp Phe Arg Asp Leu Tyr Asp Pro Arg Asp Lys Ala Lys Leu
                325                 330                 335

Leu Tyr Asn Asn Leu Asp Ala Phe Gly Ile Met Asp Tyr Thr Leu Thr
            340                 345                 350

Gly Lys Val Glu Asp Asn His Asp Asp Thr Asn Arg Ile Ile Thr Val
        355                 360                 365

Tyr Met Gly Lys Arg Pro Glu Gly Glu Asn Ala Ser Tyr His Leu Ala
    370                 375                 380

Tyr Asp Lys Asp Arg Tyr Thr Glu Glu Glu Arg Glu Val Tyr Ser Tyr
385                 390                 395                 400

Leu Arg Tyr Thr Gly Thr Pro Ile Pro Asp Asn Pro Asn Asp Lys
            405                 410                 415

<210> 2
<211> 583
<212> PRT
<213> Bos taurus

<220>
<221> PEPTIDE
<222> (1)..(583)
<223> albumin [Bos taurus].

<400> 2
Asp Thr His Lys Ser Glu Ile Ala His Arg Phe Lys Asp Leu Gly Glu
1               5                   10                  15

Glu His Phe Lys Gly Leu Val Leu Ile Ala Phe Ser Gln Tyr Leu Gln
            20                  25                  30

Gln Cys Pro Phe Asp Glu His Val Lys Leu Val Asn Glu Leu Thr Glu
        35                  40                  45

Phe Ala Lys Thr Cys Val Ala Asp Glu Ser His Ala Gly Cys Glu Lys
    50                  55                  60

Ser Leu His Thr Leu Phe Gly Asp Glu Leu Cys Lys Val Ala Ser Leu
65                  70                  75                  80

Arg Glu Thr Tyr Gly Asp Met Ala Asp Cys Cys Glu Lys Gln Glu Pro
                85                  90                  95

Glu Arg Asn Glu Cys Phe Leu Ser His Lys Asp Asp Ser Pro Asp Leu
            100                 105                 110

Pro Lys Leu Lys Pro Asp Pro Asn Thr Leu Cys Asp Glu Phe Lys Ala
        115                 120                 125

Asp Glu Lys Lys Phe Trp Gly Lys Tyr Leu Tyr Glu Ile Ala Arg Arg
    130                 135                 140

His Pro Tyr Phe Tyr Ala Pro Glu Leu Leu Tyr Tyr Ala Asn Lys Tyr
145                 150                 155                 160

Asn Gly Val Phe Gln Glu Cys Cys Gln Ala Glu Asp Lys Gly Ala Cys
                165                 170                 175

Leu Leu Pro Lys Ile Glu Thr Met Arg Glu Lys Val Leu Ala Ser Ser
            180                 185                 190

Ala Arg Gln Arg Leu Arg Cys Ala Ser Ile Gln Lys Phe Gly Glu Arg
        195                 200                 205

Ala Leu Lys Ala Trp Ser Val Ala Arg Leu Ser Gln Lys Phe Pro Lys
    210                 215                 220

Ala Glu Phe Val Glu Val Thr Lys Leu Val Thr Asp Leu Thr Lys Val

225                 230                 235                 240

His Lys Glu Cys Cys His Gly Asp Leu Leu Glu Cys Ala Asp Asp Arg
                245                 250                 255

Ala Asp Leu Ala Lys Tyr Ile Cys Asp Asn Gln Asp Thr Ile Ser Ser
            260                 265                 270

Lys Leu Lys Glu Cys Cys Asp Lys Pro Leu Leu Glu Lys Ser His Cys
        275                 280                 285

Ile Ala Glu Val Glu Lys Asp Ala Ile Pro Glu Asn Leu Pro Pro Leu
    290                 295                 300

Thr Ala Asp Phe Ala Glu Asp Lys Asp Val Cys Lys Asn Tyr Gln Glu
305                 310                 315                 320

Ala Lys Asp Ala Phe Leu Gly Ser Phe Leu Tyr Glu Tyr Ser Arg Arg
                325                 330                 335

His Pro Glu Tyr Ala Val Ser Val Leu Leu Arg Leu Ala Lys Glu Tyr
            340                 345                 350

Glu Ala Thr Leu Glu Glu Cys Cys Ala Lys Asp Asp Pro His Ala Cys
        355                 360                 365

Tyr Ser Thr Val Phe Asp Lys Leu Lys His Leu Val Asp Glu Pro Gln
    370                 375                 380

Asn Leu Ile Lys Gln Asn Cys Asp Gln Phe Glu Lys Leu Gly Glu Tyr
385                 390                 395                 400

Gly Phe Gln Asn Ala Leu Ile Val Arg Tyr Thr Arg Lys Val Pro Gln
                405                 410                 415

Val Ser Thr Pro Thr Leu Val Glu Val Ser Arg Ser Leu Gly Lys Val
            420                 425                 430

Gly Thr Arg Cys Cys Thr Lys Pro Glu Ser Glu Arg Met Pro Cys Thr
        435                 440                 445

Glu Asp Tyr Leu Ser Leu Ile Leu Asn Arg Leu Cys Val Leu His Glu
    450                 455                 460

Lys Thr Pro Val Ser Glu Lys Val Thr Lys Cys Cys Thr Glu Ser Leu
465                 470                 475                 480

Val Asn Arg Arg Pro Cys Phe Ser Ala Leu Thr Pro Asp Glu Thr Tyr
                485                 490                 495

Val Pro Lys Ala Phe Asp Glu Lys Leu Phe Thr Phe His Ala Asp Ile
            500                 505                 510

Cys Thr Leu Pro Asp Thr Glu Lys Gln Ile Lys Lys Gln Thr Ala Leu
        515                 520                 525

Val Glu Leu Leu Lys His Lys Pro Lys Ala Thr Glu Glu Gln Leu Lys
    530                 535                 540

Thr Val Met Glu Asn Phe Val Ala Phe Val Asp Lys Cys Cys Ala Ala
545                 550                 555                 560

Asp Asp Lys Glu Ala Cys Phe Ala Val Glu Gly Pro Lys Leu Val Val
                565                 570                 575

Ser Thr Gln Thr Ala Leu Ala
            580


<210> 3
<211> 166
<212> PRT
<213> Homo sapiens

<220>
<221> PEPTIDE
<222> (1)..(166)
<223> erythropoietin [Homo sapiens].

<400> 3
Ala Pro Pro Arg Leu Ile Cys Asp Ser Arg Val Leu Glu Arg Tyr Leu
1               5                   10                  15

Leu Glu Ala Lys Glu Ala Glu Asn Ile Thr Thr Gly Cys Ala Glu His
            20                  25                  30

Cys Ser Leu Asn Glu Asn Ile Thr Val Pro Asp Thr Lys Val Asn Phe
        35                  40                  45

Tyr Ala Trp Lys Arg Met Glu Val Gly Gln Gln Ala Val Glu Val Trp
    50                  55                  60

Gln Gly Leu Ala Leu Leu Ser Glu Ala Val Leu Arg Gly Gln Ala Leu
65                  70                  75                  80

Leu Val Asn Ser Ser Gln Pro Trp Glu Pro Leu Gln Leu His Val Asp
                85                  90                  95

Lys Ala Val Ser Gly Leu Arg Ser Leu Thr Thr Leu Leu Arg Ala Leu
            100                 105                 110

Gly Ala Gln Lys Glu Ala Ile Ser Pro Pro Asp Ala Ala Ser Ala Ala
        115                 120                 125

Pro Leu Arg Thr Ile Thr Ala Asp Thr Phe Arg Lys Leu Phe Arg Val
    130                 135                 140

Tyr Ser Asn Phe Leu Arg Gly Lys Leu Lys Leu Tyr Thr Gly Glu Ala
145                 150                 155                 160

Cys Arg Thr Gly Asp Arg
                165

<210> 4
<211> 129
<212> PRT
<213> Gallus gallus

<220>
<221> PEPTIDE
<222> (1)..(129)
<223> egg white lysozyme [Gallus gallus].

<400> 4

<210> 5
<211> 385
<212> PRT

<213> Gallus gallus

<220>
<221> PEPTIDE
<222> (1)..(385)
<223> Ovalbumin (Egg Albumin).

<400> 5
Gly Ser Ile Gly Ala Ala Ser Met Glu Phe Cys Phe Asp Val Phe Lys
1               5                   10                  15

Glu Leu Lys Val His His Ala Asn Glu Asn Ile Phe Tyr Cys Pro Ile
            20                  25                  30

Ala Ile Met Ser Ala Leu Ala Met Val Tyr Leu Gly Ala Lys Asp Ser
        35                  40                  45

Thr Arg Thr Gln Ile Asn Lys Val Val Arg Phe Asp Lys Leu Pro Gly
    50                  55                  60

Phe Gly Asp Ser Ile Glu Ala Gln Cys Gly Thr Ser Val Asn Val His
65                  70                  75                  80

Ser Ser Leu Arg Asp Ile Leu Asn Gln Ile Thr Lys Pro Asn Asp Val
                85                  90                  95

Tyr Ser Phe Ser Leu Ala Ser Arg Leu Tyr Ala Glu Glu Arg Tyr Pro
            100                 105                 110

Ile Leu Pro Glu Tyr Leu Gln Cys Val Lys Glu Leu Tyr Arg Gly Gly
        115                 120                 125

Leu Glu Pro Ile Asn Phe Gln Thr Ala Ala Asp Gln Ala Arg Glu Leu
    130                 135                 140

Ile Asn Ser Trp Val Glu Ser Gln Thr Asn Gly Ile Ile Arg Asn Val

145                 150                 155                 160

Leu Gln Pro Ser Ser Val Asp Ser Gln Thr Ala Met Val Leu Val Asn
                165                 170                 175

Ala Ile Val Phe Lys Gly Leu Trp Glu Lys Ala Phe Lys Asp Glu Asp
            180                 185                 190

Thr Gln Ala Met Pro Phe Arg Val Thr Glu Gln Glu Ser Lys Pro Val
        195                 200                 205

Gln Met Met Tyr Gln Ile Gly Leu Phe Arg Val Ala Ser Met Ala Ser
    210                 215                 220

Glu Lys Met Lys Ile Leu Glu Leu Pro Phe Ala Ser Gly Thr Met Ser
225                 230                 235                 240

Met Leu Val Leu Leu Pro Asp Glu Val Ser Gly Leu Glu Gln Leu Glu
                245                 250                 255

Ser Ile Ile Asn Phe Glu Lys Leu Thr Glu Trp Thr Ser Ser Asn Val
            260                 265                 270

Met Glu Glu Arg Lys Ile Lys Val Tyr Leu Pro Arg Met Lys Met Glu
        275                 280                 285

Glu Lys Tyr Asn Leu Thr Ser Val Leu Met Ala Met Gly Ile Thr Asp
    290                 295                 300

Val Phe Ser Ser Ser Ala Asn Leu Ser Gly Ile Ser Ser Ala Glu Ser
305                 310                 315                 320

Leu Lys Ile Ser Gln Ala Val His Ala Ala His Ala Glu Ile Asn Glu
                325                 330                 335

Ala Gly Arg Glu Val Val Gly Ser Ala Glu Ala Gly Val Asp Ala Ala
            340                 345                 350

Ser Val Ser Glu Glu Phe Arg Ala Asp His Pro Phe Leu Phe Cys Ile
        355                 360                 365

Lys His Ile Ala Thr Asn Ala Val Leu Phe Phe Gly Arg Cys Val Ser
    370                 375                 380

Pro
385

<210> 6
<211> 594
<212> PRT

<213> Neisseria meningitidis

<220>
<22 l> PEPTIDE
<222> (1)..(594)
<223> >gi|458843|emb|CAA54878.1| outer membrane protein
p64k or PM-6 [Neisseria meningitidis]

<400> 6
Met Ala Leu Val Glu Leu Lys Val Pro Asp Ile Gly Gly His Glu Asn
1               5                   10                  15

Val Asp Ile Ile Ala Val Glu Val Asn Val Gly Asp Thr Ile Ala Val
            20                  25                  30

Asp Asp Thr Leu Ile Thr Leu Glu Thr Asp Lys Ala Thr Met Asp Val
        35                  40                  45

Pro Ala Glu Val Ala Gly Val Val Lys Glu Val Lys Val Lys Val Gly
    50                  55                  60

Asp Lys Ile Ser Glu Gly Gly Leu Ile Val Val Val Glu Ala Glu Gly
65                  70                  75                  80

Thr Ala Ala Ala Pro Lys Ala Glu Ala Ala Ala Ala Pro Ala Gln Glu
                85                  90                  95

Ala Pro Lys Ala Ala Ala Pro Ala Pro Gln Ala Ala Gln Phe Gly Gly
            100                 105                 110

Ser Ala Asp Ala Glu Tyr Asp Val Val Val Leu Gly Gly Gly Pro Gly
        115                 120                 125

Gly Tyr Ser Ala Ala Phe Ala Ala Ala Asp Glu Gly Leu Lys Val Ala
    130                 135                 140

Ile Val Glu Arg Tyr Lys Thr Leu Gly Gly Val Cys Leu Asn Val Gly
145                 150                 155                 160

Cys Ile Pro Ser Lys Ala Leu Leu His Asn Ala Ala Val Ile Asp Glu
                165                 170                 175

Val Arg His Leu Ala Ala Asn Gly Ile Lys Tyr Pro Glu Pro Glu Leu
            180                 185                 190

Asp Ile Asp Met Leu Arg Ala Tyr Lys Asp Gly Val Val Ser Arg Leu
        195                 200                 205

Thr Gly Gly Leu Ala Gly Met Ala Lys Ser Arg Lys Val Asp Val Ile
    210                 215                 220

Gln Gly Asp Gly Gln Phe Leu Asp Pro His His Leu Glu Val Ser Leu
225                 230                 235                 240

Thr Ala Gly Asp Ala Tyr Glu Gln Ala Ala Pro Thr Gly Glu Lys Lys
            245                 250                 255

Ile Val Ala Phe Lys Asn Cys Ile Ile Ala Ala Gly Ser Arg Val Thr
        260                 265                 270

Lys Leu Pro Phe Ile Pro Glu Asp Pro Arg Ile Ile Asp Ser Ser Gly
    275                 280                 285

Ala Leu Ala Leu Lys Glu Val Pro Gly Lys Leu Leu Ile Ile Gly Gly
   290                 295                 300

Gly Ile Ile Gly Leu Glu Met Gly Thr Val Tyr Ser Thr Leu Gly Ser
305                 310                 315                 320

Arg Leu Asp Val Val Glu Met Met Asp Gly Leu Met Gln Gly Ala Asp
            325                 330                 335

Arg Asp Leu Val Lys Val Trp Gln Lys Gln Asn Glu Tyr Arg Phe Asp
        340                 345                 350

Asn Ile Met Val Asn Thr Lys Thr Val Ala Val Glu Pro Lys Glu Asp
    355                 360                 365

Gly Val Tyr Val Thr Phe Glu Gly Ala Asn Ala Pro Lys Glu Pro Gln
   370                 375                 380

Arg Tyr Asp Ala Val Leu Val Ala Ala Gly Arg Ala Pro Asn Gly Lys
385                 390                 395                 400

Leu Ile Ser Ala Glu Lys Ala Gly Val Ala Val Thr Asp Arg Gly Phe
            405                 410                 415

Ile Glu Val Asp Lys Gln Met Arg Thr Asn Val Pro His Ile Tyr Ala
        420                 425                 430

Ile Gly Asp Ile Val Gly Gln Pro Met Leu Ala His Lys Ala Val His
    435                 440                 445

Glu Gly His Val Ala Ala Glu Asn Cys Ala Gly His Lys Ala Tyr Phe
   450                 455                 460

Asp Ala Arg Val Ile Pro Gly Val Ala Tyr Thr Ser Pro Glu Val Ala
465                 470                 475                 480

Trp Val Gly Glu Thr Glu Leu Ser Ala Lys Ala Ser Gly Arg Lys Ile
            485                 490                 495

Thr Lys Ala Asn Phe Pro Trp Ala Ala Ser Gly Arg Ala Ile Ala Asn
            500                 505                 510

Gly Cys Asp Lys Pro Phe Thr Lys Leu Ile Phe Asp Ala Glu Thr Gly
        515                 520                 525

Arg Ile Ile Gly Gly Gly Ile Val Gly Pro Asn Gly Gly Asp Met Ile
    530                 535                 540

Gly Glu Val Cys Leu Ala Ile Glu Met Gly Cys Asp Ala Ala Asp Ile
545                 550                 555                 560

Gly Lys Thr Ile His Pro His Pro Thr Leu Gly Glu Ser Ile Gly Met
                565                 570                 575

Ala Ala Glu Val Ala Leu Gly Thr Cys Thr Asp Leu Pro Pro Gln Lys
            580                 585                 590

Lys Lys

## Description

### DESCRIPTIVE MEMMORY:

The present invention is related with the field of the biotecology particularly in the field of proteomics. The proteomics is a discipline that comprises an entire group of analytical tools and procedures whose object of study is the proteome, which is defined as the protein complement of the total group of genes (genome) of an organism.
The proteome of an organism is very dynamic and although it is possible to predict the proteins that are derived of a genome it is impossible to predict the proteins that are expressing in a given moment and its respective relative quantities (quantitative proteomics). The proteomics is today a reality thanks to the harmonious combination of two analytical tools: the two-dimensional electrophoresis (2DE) and the mass spectrometry (MS). The 2DE are the most powerful tool for the separation of complex mixtures of proteins and it allows their relative quantification. The mass spectrometry on the other hand, possesses a very high sensitivity (fmoles), it allows the structural elucidation of the analyzed peptides and the detection of postraslational modifications.
The 2DE possess several limitations (Membrane proteins and proteomics: An impossible amour. Santoni V., Molloy M., Rabilloud T. Electrophoresis. 21, 1054-1070, 2000; Proteome profiling-pitfall and progress. Haynes P.A., Yachts III J.R. Yeast. 17, 81-87, 2000) that impede their usage in a massive scale in proteomics studies:
- Difficulties in the analysis of hydrophobic proteins. For example, it is well-known that the membrane proteins that are always attractive candidates for the vaccine development are under-represented in two-dimensional gels.
- Proteins of extreme isoelectric points can not be focalized efficiently.
- The elaboration of high quality and reproducible two-dimensional gels require a great laboriousness and manual skills.
- It is not coupled directly to the mass spectrometers therefore it limits considerably the high throughput analysis in a rasonable time.
- The images analysis of gels in an automatic way to detect proteins differentially expressed is not the enough efficient to obviate the specialist intervention and this is time-consuming.
For these reasons, has merged a trend that prefers to carry out the quantitative proteomic studies with peptides instead of carrying out it with intact proteins. This is possible thanks to that a short sequence of peptides (3 to 5 amino acids) determined by mass spectrometry is enough to perform a reliable identification of the protein that originated it, in the sequence databases (Error-tolerant identification of peptides in sequence databases by peptide sequence tags. Mann M, Wilm M. Anal. Chem. 66, 4390-4399, 1994). On the other hand, the proteolytic digestion of a hydrophobic protein can even generate some non-hydrophobics peptides which are easily handled. These reasons have also favored that strategies arise to carry out studies of high throughput quantitative proteomics by identifying a great number of proteins and do not use the two-dimensional electrophoresis.
The pioneering work in this address was done in 1999 by Link and coworkers. (Link, A.J. et al. Direct analysis of protein complexes using mass spectrometry. Nat. Biotechnol. 17, 676-682, 1999), when they developed a two-dimensional chromatografic system coupled directly to mass spectrometry (LC-MS/MS). These authors packed a microcapilar cation exchange column followed by a reverse phase column. The peptides are injected at acidic pH and all are retained in the cation exchanger, later on, by increasing the saline concentration of the mobile phase, a fraction of these peptides is eluted onto the reverse phase column. A continuous gradient of acetonitrile elutes the peptides retained in the reverse phase column and they are analyzed by mass spectrometry to proceed to their identification in the sequence databases. This process is repeated several times until the complete elution of all the peptides retained in the of cation exchange column. In the scientific literature this procedure is known as MudPiT (Multidimensional Technology for the Identification of Proteins), and it has been able to identify 1484 proteins starting from the hydrolizate of the total proteins of *Saccharomyces cerevisiae* (Washburn M. P. et al. Large-scale analysis of the yeast proteome by multidimensional protein identification technology, Nature Biotechnology 19, 242-247, 2001).
The current experience demonstrates that this fractionation is essential for identifying a great number of proteins since the efficiency of the current mass spectrometers and chromatography systems do not allow a direct analysis of the complex mixtures of peptides generated by the proteolysis of a particular proteome. This procedure undoubtely impelled the highthroughput protein identification when coupling on-line the chromatography systems to the mass spectrometers, however, it was not still solved the determination of the relative quantification of proteins present in the mixtures under study. The first step to solve this problem was carried out by Washburn MP et al. (Analysis of quantitative proteomic dates generated via multidimensional protein identification technology. Anal. Chem. 74:1650-1656, 2002), when growing cells of *Saccharomyces cerevisiae* in a culture media containing nitrogen-14 (¹⁴N) and other cells in a media enriched with nitrogen-15 (¹⁵N). When carrying out this procedure all the derived proteins from one condition were labeled with ¹⁵N and those of the other condition with ¹⁴N. They mixed up the proteins obtained under both conditions; the specific proteolysis of the total proteins was made and preceded to the identification of the proteins by peptide sequencing by mass spectrometry. The determination of the relative quantities of the proteins is carried out by the intensities ratio of the peptides labeled with ¹⁵N/¹⁴N.
However, this strategy of isotopic labeling is not always possible to carry out, it being alone viable at the moment in simple organisms as yeasts and bacterias due to the high cost of the istopically enriched cultivation media. On the other hand, this type of labeling is not always feasible in the biological systems under study and it is necessary to poit out that the labeling with ¹⁵N introduces certain complications in the identification process since a mass shift introduced between the labeled and the non-labeled peptide depends on the number of nitrogen atoms present in the peptide sequence. To overcome this last obstacle other authors restricted the introduction of the isotopic labeling to some amino acids when growing the organism under study in culture media suplemented with a particular labeled amino acid. This strategy was denominated as SILAC (stable isotope labeling by amino acids in cell culture) and there is a considerable number of publications that have used the labeling with ¹²C/¹³C and ¹H/²H using culture media suplemented with labeled and non-labeled leucine, or lysine in the two compared conditions (S.E. Ong, B. Blagoev, I. Kratchmarova, D.B. Kristensen, H. Steen, A. Pandey, M. Mann, Mol. Cell Proteomics 2002, 1, 376-386; Berger SJ, Lee SW, Anderson GA, Lijiana PT, Tolic N, Shen E, Zhao R, Smith RD Global High-throughput Peptide Proteomic Analysis by Combining Stable Isotope Amino Acid Labeling and Date-Dependent Multiplexed-MS/MS. Analytical Chemistry 2002, 74, 4994-5000; and Precise peptide sequencing and protein quantification in the human proteome through in vivo lysine-Specific Mass Tagging, J. Am. Soc. Mass Spectrom. 2003, 14, 1-7). The mass shift of the light-peptide with respect to the heavy one only happens if the peptide sequence contains the labeled amino acid; therefore peptides that do not carry the labeled amino acid cannot be used in the quantification. The usage of SILAC can not be used in a universal way in all the proteomics experiments for its high cost and it is only applicable to biological problems that are studied under culture media.
The labeling with ¹⁸O is a more universal method to carry out the quantification in the proteomic experiments because it can be incorporated in the C-terminal carboxyl groups of all peptides generated by the proteolysis of a complex mixture of proteins. One of the protein mixtures is digested in presence of a buffer prepared in normal water while the other is digested in presence of water labeled with ¹⁸O (H₂¹⁸O). The peptides that are obtained in a buffer prepared with H₂¹⁸O can incorporate one and two atoms of ¹⁸O in their C-terminal end, on the other hand, the other peptides shows their natural isotopic distribution. To proceed to the relative quantification of the labeled and non-labeled species it is necessary to calculate the areas corresponding to the isotopic ditributions of the species labeled with ¹⁶O/¹⁸O in the mass spectrum and once peptides have been identified, infering the proportions of the proteins that contained them. This type of labeling present two limitations, on one hand, an enough separation does not take place among the isotopic distributions of the labeled and non-labeled peptides and on the other hand, the addition of ¹⁸O is not homogeneous adding one and two atoms of ¹⁸O for each peptide. These two problems can have serious implications in the relative quantification of the light- and heavy-species unless appropriate software is capable to interpret correctly the complex overlaped the isotopic distributions.
To avoid the overlapping of the isotopic distributions and to homogenize the incorporation of ¹⁸O in the C-terminal end of péptidos, Yao and coworkers (Yao X, Afonso C and Fenselau C. Dissection of proteolytic 180 labeling: endoprotease-catalyzed 16O-to-18O exchange of truncated peptide substrates. J. Proteome Res. 2003, 2,147-52) proposed the complete labeling with ¹⁸O of proteolytic peptides after the digestion process by means of the long incubation of the proteolytic peptides in the presence of trypsin.
By this procedure, they outlined that the complete incorporation of two atoms of ¹⁸O is guaranteed at the C-terminal end of the tryptic peptides and a separation of 4 Da is achieved between the isotopic distributions of the labeled and non-labeled peptides. However, there are peptides that are resistant to the incorporation of ¹⁸O-atoms because this process possesses a very inferior affinity in comparison with that of the trypsin cleavage of the peptide bonds at the C- terminal end of the basic aminoacids lysine and arginine. If this happen, big errors can be introduced in the quantification. On the other hand, the long incubation times of tryptic peptides to guarantee the complete exchange of ¹⁶O by ¹⁸O can facilitate the emergence of non-specific cleavages in the sequences and it affect the results of the identification in proteins databases.
The labeling with ¹⁸O but in an specific mode (González J, Takao T, Hori H, Besada V, Rodriguez R, Padron G, Shimonishi Y. A Method for Determination of N-Glycosylation Sites in Glicoproteins by Collision-Induced Dissociation Analysis in Fast Atom Bombardment Mass Spectrometry: Identification of the Positions of Carbohydrate-Linked Asparagine in Recombinant - Amylase by treatment with PNGase-F in 18O-labeled Water. Anal. Biochem., 1992, 205, 151-158) has also been used in the proteomics to identify the N-glycopeptides in a reliable way in the databases of sequences and to discriminate false positive (Kuster, B and Mann M. 18O-labeling of N-glycosylation sites to improve the identification of gel-separated glycoproteins using peptide mass mapping and database searching. Anal. Chem. 1999, 71, 1431-1440). The glycoproteins or glycopeptides are deglycosylated with the PNGase-F in presence of a buffer prepared with ¹⁸O-labeled water and the N-glycosylated asparagine residues (Asn-X-Ser/Thr) are transformed in aspartic acid residues that incorporate in a highly specific way at their side chain an atom of ¹⁸O. In the quantitative proteomics this same procedure can be used to the deglycosylate the glycoproteins of one condition in a buffer prepared with normal water and the glycoproteins of the other condition in presence of ¹⁸O-labeled water.
After mixing equals proportions of the analyzed samples, the relative quantities of each protein are estimated in a same way as the ratio of ¹⁶O/¹⁸O (González J, Takao T, Hori H, Besada V, Rodriguez R, Padron G, Shimonishi Y. A Method for Determination of N-Glycosylation Sites in Glycoproteins by Collision - Induced Dissociation Analysis in Fast Atom Bombardment Mass Spectrometry: Identification of the Positions of Carbohydrate-Linked Asparagine in Recombinant-Amylase by treatment with PNGase-F in 18O-labeled Water. Anal. Biochem. 1992, 205, 151-158).
Another way of introducing the labeling in all the proteolytic peptides to make quantitative proteomics was introduced by Zappacosta and Annan (Zappacosta F, and Annan RS. N-terminal isotope tagging strategy for quantitative proteomics: results-driven analysis of protein abundance changes. Anal Chem. 2004, 76, 6618-6627). In a first step, all the lysine residues are transformed as homoarginine by a reaction with O-methyl isourea and later on, all the amino terminal groups of all proteolytic peptides obtained in one of the compared conditions are derivatized with a blocking agent enriched in heavy isotopes (particularly deuterium) while the peptides obtained in the other condition are modified with the non-labeled reagent. Both samples are mixed up and proceed to the relative quantification when estimating the proportion of the intensities of the isotopic distributions of the heavy and light-peptides.
To avoid the overlapping of the isotopic distributions of the labeled and non-labeled species the derivatization of the peptides is made with deuterated propionic anhydride (d₅) and with normal propionic anhydride. However, the incorporation of more than 3 deuterium atoms can introduce errors in the relative quantification of the light- and heavy-species because their retention times in reverse phase chromatography are different as demonstrated by Zhang and coworkers ( Zhang R, Sioma CS, Wang S, Regnier FE.Fractionation of isotopically labeled peptides in quantitative proteomics. Anal Chem. 2001, 73, 5142-5149). The errors in the quantification can increase in the same measure that the quantity of deuterium atoms increases in the sequence of one of the species (Zhang R, Sioma CS, Thompson RA, Xiong L, Regnier FE. Controlling deuterium isotope effects in comparative proteomics. Anal Chem. 2002; 74, 3662-3669) and it has been seen that when the derivatization is carry out with the same blocking reagent but labeled with ¹³C these errors they are minimized (Zhang R, Regnier FE, Minimizing resolution of isotopically coded peptides in comparative proteomics. J. Proteome Res. 2002, 1,139-147).
The analysis of the proteolysis of complex mixtures of proteins constitutes a great challenge because the overwhelming number of the generated peptides surpasses the resolution power of the current chromatography systems and the most modern mass spectrometers.
To approach this challenge and be able to carry out the quantitative proteemics without the necessity of using the two-dimensional electrophoresis, a current tendency has been the simplification of the peptide mixture by developing methods that allow the selective isolation of a subset of peptides that possess a common characteristic, and that its study does not affect the representativeness of the proteins that originated them, that is to say, it ispossible to characterize a bigger number of proteins present in the initial mixture. The combination of a selective isolation of peptides with appropriate isotope labeling techniques not only allows the identification but also the relative quantification of the proteins present in the compared initial mixtures.

### Selective isolation of cysteine containing peptides

This approach was begun by Gigy and coworkers (Quantitative analysis of complex protein mixes using isotope-coded affinity tags. Gygi, S. P., Rist, B., Gerber, S. A., Turecek, F., Gelb, M. H., and Aebersold, R. Nat. Biotechnol. 1999, 17, 994-999) when proposing the well-known ICAT method (isotope-code affinity tags) that is based in the selective isolation of cystein-containing peptides. The method combines the affinity chromatography (streptavidin-biotin) and the labeling with the ICAT reagent in its light and heavy variants. This reagent consists of three functional elements:
1 - a group that reacts specifically with the thiol groups of the cystein residues.
2 - a group that has an affinity element (biotin) and allows the selective isolation of the peptides that react with ICAT reagent.
3 - an arm that separates the elements mentioned above. In the heavy variant it has 8 deuterium atoms in its structure (heavy ICAT) and the light variant has 8 atoms of hydrogen (light ICAT).

Once the proteins coming from both conditions are separately reduced in presence of DTT, the free cysteines generated in one of the conditions react with the heavy ICAT and those generated in the other condition with the light ICAT. Both proteins mixtures are joined in identical quantities and the proteolytic digestion is performed. The generated peptides are purified by a streptavidine affinity column and as a consequence the cysteine containing peptides modified with the ICAT reagents are isolated selectively.

To proceed to the relative quantification, the relative intensities of the signals corresponding to the peptides labeled with the light- and heavy-ICAT are measured. The masses of the peptides labeled with these reagents differ in multiples of 8 units of masses, depending the number of cysteine residues contained in the sequence.

This methodology presents several limitations:
- The size of the ICAT reagent is considerable and it can affect the ionization efficiency of peptides and the interpretation of the mass spectra.
- The presence of 8 deuterium atoms in the peptide modified with the heavy ICAT can cause big errors in the quantification because its retention time can differ considerably respect to the peptide modified with the light ICAT (Zhang R, Sioma CS, Wang S, Regnier F. Fractionation of isotopically labeled peptides in quantitative proteomics. Anal Chem. 2001, 73, 5142-5149) and the intensities ratios are not the same during the elution time of the light and heavy species of the peptide to be quantified.
- The quantification procedure described for the ICAT is not applicable to other methods of isotopic labeling that do not separate enogh the signals to avoid the overlapping of its isotopic distributions.
- If it is desirable to fractionate the peptide mixture additionally as the case of MudPit, (Washburn M. P. et al. Large-scale analysis of the yeast proteome by multidimensional protein identification technology, Nature Biotechnology, 2001, 19, 242-247) it is necessary to use a chromatography different to the used for the selective isolation of peptides and it causes considerable losses in the manipulation of the samples.
- When using a high affinity chromatography during the selective isolation step, the losses can be considerable for some particular peptides.
- Proteins without cysteines cannot be analyzed by this method.

### Selective isolation of methionine containing peptides.

Recently it has been described in the literature a method named as COFRADIC that allows the selective isolation of methionine-containing peptides (Gevaert K, Van Damme J, Goethals M, Thomas GR, Hoorelbeke B, Demol H, Martens L, Puype M, Staes A, Vandekerckhove J. Chromatographic isolation of methionine-containing peptides for gel-free proteome analysis: identification of lives than 800 Escherichia coli proteins. Mol Cell Proteomics. 2002, 11, 896-903). This method consists of several steps: after reducing and S-alkylating all proteins, they are digested and the resultant peptides are fractioned by reverse phase chromatography and collected in a considerable number of fractions in what the authors denominate as a primary run. Each one of these fractions reacts independently with a solution of hydrogen peroxide (3% v/v) during 3 minutes, and they are analyzed again in the same chromatography system under the same conditions in a second run. The peptides that contain metionine are isolated selectively because once oxydized they become less-hydrophobic species and decrease their retention times so they differ from the rest of the peptides that do not contain methionine whose retention times remain invariable and they are discarded. The authors outlined that this method allows a simplification of the complex mixture of péptidos in an similar extension to the obtained by the ICAT and it can also be applied to the selective isolation of fosfopéptidos, N-terminal peptides of proteins, peptidos linked by disulfide bridges (Martens L, Van Damme P, Damme JV, Staes A, Timmerman and, Ghesquiere B, Thomas GR, Vandekerckhove J, Gevaert K. The human platelet proteome mapped by peptide-centric proteomics: To functional protein profile. Proteomics. 2005, 5(12):3193-204.
Although the authors outlined that the oxidation conditions have been optimized to avoid the oxidation of labile residues like cystein and tryptophan, if this happened the selectivity of the method it would be affected and the degree of simplification of the peptide mixture that would be reached would not be similar to that of the ICAT method as the authors claim.
On the other hand, although the method can be automated to achieve the selective isolation of all the methionine containing peptides it is necessary to carry out a great number of chromatographic runs and the global yield of the method can in turns be affected.

### Selective isolation of the N-terminal peptides.

A variant of the COFRADIC has also been proposed to isolate selectively the N-terminal peptide of all the proteins (Gevaert K, Goethals M, Martens L, Van Damme J, Staes TO, Thomas GR, Vandekerckhove J. Exploring proteomes and analyzing protein processing by mass spectrometric identification of sorted N-terminal peptides. Nat Biotechnol. 2003; 21, 566-569). The first step of this method consists in the blockage of all primary amino groups of the proteins present in the compared complex mixtures, then a specific proteolysis of the modified proteins is performed and by reverse phase chromatography the peptide mixture is separated in a considerable number of fractions.
The new amino groups of the internal peptides generated during the proteolysis that are present in each one of these fractions react additionally with a highly hydrophobic blocking group and again are separated in the same chromatographic system under conditions identical to that of previously mentioned. The retention time of all internal peptides is increased considerably by the additions of the second blocking reagent, however all the N-terminal peptides that were blocked in the first step by this way are isolated selectively when being collected in the same time of retention of its original fraction. This strategy can have as disadvantage: to perform a reliable quantification the first step consiting the blockage of the aminos groups should work in a quantitative way and this can be something difficult to achieve when proteins are present in complex mixtures.
Also in this first step, the blocking of amino groups can diminish the solubility of the proteins considerably and it can originate precipitations that can affect the quantitativity of the method. Lastly, the fact that a single peptide per protein is isolated is an excessive simplification and it may have a negative impact in the identification and the quantification of the present proteins in the complex mixtures. A method that allows redundancy by isolating a reduced group of 3-4 peptide per proteins can be ideal for proteomics studies without the usage of the two-dimensional electrophoresis since it permits the confirmation of the quantification results.

### Selective isolation of N-glycopeptides.

It is reported that approximately 91 percent of the membrane proteins reported in the Swissprot databases are glycoproteins. It has been proposed a strategy based on the selective isolation of glycopeptides by using lectin affinity chromatgraphy for proteomics studies (Geng M, Zhang X, Bina M, Regnier F, Proteomics of glycoproteins based on affinity selection of glycopeptides from tryptic digests. J. Chromatogr. B. Biomed. Sci. Appl. 2001, 752, 293-306; Kaji H, Saito H, Yamauchi Y, Shinkawa T, Taoka M, Hirabayashi J, Kasai K, Takahashi N, Isobe T. Lectin affinity capture, isotope-coded tagging and mass spectrometry to identify N-linked glycoproteins. Nat Biotechnol. 2003, 21, 667-672). Te usage of a particular lectin or a battery of them (Ji J, Chakraborty A, Geng M, Zhang X, Amini A, Bina M, Regnier F. Strategy for qualitative and quantitative analysis in proteomics based on signature peptides. J. Chromatogr. B Biomed. Sci. Appl. 2000, 745, 197-210, Geng M, Ji J, Regnier FE, Signature-peptide approach to detecting proteins in complex mixes. J. Chromatogr. A. 2000, 870(1-2), 295-313), immobilized in a chromatographic column, possesses a limitation because they are not able to recognize all the existent glycoforms and to guarantee an efficient selective isolation. To overcome this limitation Zhang and collaborators (Zhang H, Li XJ, Martin DB, Aebersold R. Identification and quantification of N-linked glycoproteins using hydrazide chemistry, stable isotope labeling and mass spectrometry. Nat Biotechnol. 2003, 21, 627-629) proposed the immobilization of the glycoproteins to a solid support by means of the derivatization with hydrazine and later on, their releasing by the action of the PNGase-F. This last step is carried out in the presence and absence of H₂¹⁸O allowing the relative quantification of the proteins expressed under two biological conditions. This method can be applied to samples of biological interest such as membrane proteins which are considered as a vaccine candidates or receptors or to serum that is the most complex proteoma that exists, however its applicability it is restricted only to those samples that are enriched in glycoproteins.

### Selective isolation of peptides with histidine residues.

The abundance of the histidine in the human proteome is similar to that of the cystein. The hisitidine is present in 83% of the proteins of the human proteome and 15% of the tryptic peptides contain this amino acid (Regnier FE, Riggs L, Zhang R, Xiong L, Liu P, Chakraborty E, Seeley E, Sioma C, and Thompson RA. J. Mass Spectrom. 2002, 37, 133-145). For these reasons, the selective isolation of peptides with histidine is attractive to achieve a considerable simplification of complex mixtures of peptides for proteomics studies. The affinity chromatography has been used for the selective capture of peptides with histidine by the formation of a coordination complex with the metal ions immobilized in the chromatographic support. There are several works that evaluate different matrices and the immobilized metals (Ren D, Penner NA, Slentz BE, Inerowicz HD, Rybalko M, Regnier FE. Contributions of commercial solvents to the selectivity in immobilized metal affinity chromatography with Cu(II). J Chromatogr A. 2004, 1031, 87-92) but the results demonstrate that the specificity is still inferior compared with that of the other previously described methods for the selective isolation ( Ren D, Penner NA, Slentz BE, Mirzaei H, Regnier F. Evaluating immobilized metal affinity chromatography for the selection of histidine-containing peptides in comparative proteomics. J Proteome Res. 2003, 2, 321-329; Ren D, Penner NA, Slentz BE, Regnier FE. Histidine-rich peptide selection and quantification in targeted proteomics. J. Proteome Res. 2004, 3, 37-45). In fact, variants have been explored including the previous modification of peptides to increase the specificity.

### Isolation of peptides with arginine at the C-terminal end.

Recently, Foettinger and coworkers (Foettinger A., Leitner A., Lindner W. Solid-phase captures and release of arginine peptides by selective tagging and boronate affinity chromatography. J. Chrom. A. 2005, 1079, 187-196) proposed a method for the capture in solid phase of peptides that contain arginine by using boronate affinity chromatography columns. In this work, the authors outlined that these peptides can be released efficiently before being analyzed by mass spectrometry. The method is based on the selective and covalent modification of the guanidinium group of arginine with 2,3-butanodione under alkaline conditions (pH>8) and the retention of the modified peptides in a solid support with immobilized phenylboronic acid. The peptides that do not contain arginine are discarded in the non-retained fraction and those that contain arginine can be eluted from the solid support at acidic pH thanks to the reversibility of the reactions previusly described. This method possesses some disadvantages that are necessary to highlight because in some cases it compromise its applicability in proteomics studies. On one hand, the affinity selection this chromatography can have non-specific binding of peptides with lysin resides and their magnitude oscillates (10-90 %). On the other hand, the simplification grade (approximately 50%) that would be achieved it is insufficient since the arginine is an abundant amino acid in all the genomes sequenced so far, and therefore even after the boronate affinity chromatography the sample to analyze continues being very complex.

### Selective isolation of peptides with serine and threonine at the N - terminal end.

In the 2003 Chelius and Shaler (Chelius D, Shaler TA. Capture of peptides with N-terminal serine and threonine: A sequence-specific chemical method for peptide mixtures simplification. Bioconjugate Chem. 2003, 14, 205-211) described a method for the selective isolation of peptides that possess serine and threonine in their N -terminal end. The method consists of several steps (1) conversion of the hydroxyl groups of serine and threonine located at the N -terminal end of peptides to carbonyl groups by means of an oxydative treatment with periodate; (2) reaction of the new formed carbonyl group with substituted hidrazides to form hydrazones and (3) selective isolation of the labeled peptides by using affinity chromatography. In this work the authors propose the labeling of the peptides that possess serine and threonine in the N -terminal end with biotin for their later selective isolation using beads with immobilized streptavidine. This method possesses several disadvantages: (a) the step of oxydation with periodate introduces other chemical modifications in the peptides like the oxydation of all methionine residues, (b) the elution step of peptides from the affinity column by a treatment at acidic pH introduces degradation in the linker of the modified peptides. In this work Chelius D, Shaler TA. Capture of peptides with N-terminal serine and threonine: A sequence-specific chemical method for peptide mixtures simplification. Bioconjugate Chem. 2003, 14, 205-211) does not appear an application of this method to the characterization of the complex of mixtures of proteins.

### Selective isolation of peptides by cation exchange chromatography.

The cation exchange chromatography has also been used by Betancourt and coworkers (SCAPE: A new tool for the Selective Captures of Peptides in Protein identification. Betancourt L, Gil J, Besada V, González LJ, Fernández-de-Cossio J, Garcia L, Pajón R, Sánchez A, Alvarez F, Census G. J. Proteome Res. 2005, 4, 491-496) in proteomics studies when isolating selectively peptides that do not contain neither histidine nor arginine (nHnR peptides) in their sequences. This method is based on the combination of the reversible blocking of amino groups and the usage of a chromatography system that it is able to separate in an effective and simple way the positively-charged peptides (charge 1+, 2+ ,3+, 4+, etc) from the neutral peptides (charge zero). This method achieves a considerable simplification of the analyzed peptide mixture in a similar extension to the one achieved with the ICAT (Quantitative analysis of complex protein mixes using isotope-coded affinity tags. Gygi, S. P., Rist, B., Gerber, S. A., Turecek, F., Gelb, M. H., and Aebersold, R. Nat. Biotechnol. 17, 994-999, 1999).
After the blocking reaction of the amino groups this chromatgraphic system retains the charged peptides containing most of them arginine and histidine, while in the non-retained fraction are the neutral peptides that do not contain neither histidine nor arginine within their sequences (nHnR peptides). Before analyzing in the mass spectrometer the nHnR peptides, the blocking group that they possess is eliminated by means of a hydrolytic treatment to regenerate the free amino groups and make more favorable its ionization, fragmentation in mass spectrometric analysis and consequently its identification in the databases.
This method isolates the nHnR peptides in the non-retained fraction of the cation exchange chromatography and to achieve the identification of a bigger number of proteins it requires other chromatographic steps for its further fractionation. These additional chromatgraphic steps can cause losses during the manipulation and affect the yields of the method. On the other hand, the treatments of unblocking of the amino groups of peptides can cause degradations and to affect considerably the recovering of some particular peptides.
Due to the limitations described for these methods of selective isolation of peptides it continues being very necessary to identify and to determine levels of expression of the proteins presentin complex mixtures, through the selective and specific isolation of a small group of peptides by means of the simplification of the complex mixture to be characterized before their mass spectrometric analysis.

### DETAILED DESCRIPTION OF THE INVENTION

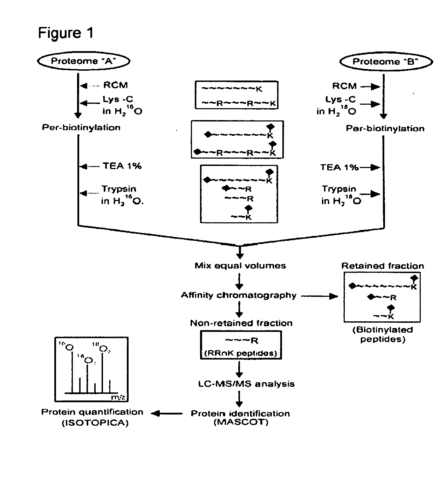

The method of selective isolation of peptides proposed in the present invention achieves the simplification of the complex mixture of proteolytic peptides by isolating in an effective and simple way in the non-retained fraction of the chromatographic step those peptides that: (1) do not possess any modifier of amino group , (2) they were originated by the cleavage of the trypsin at the C-terminal end of arginine residues and (3) they do not have any lysine residue within their sequence (RRnK peptides).
This method can be used for the identification of the constituent proteins of complex mixtures and for the determination of their relative quantities under the compared conditions. For this purpose, the mixture of proteins obtained either artifitially or from natural source is treated according to the steps that are described in the figure 1 and are explained below:
***(1) Reduction and S-alkylation of cyteine residues*** with any of the reagents used forthis purpose, for example iodoacetamide, iodoacetic acid, acrylamide, 4-vinylpiridine, etc. This initial step is of a great importance due to several reasons: (a) it assures a bigger efficiency of the next step of the enzymatic hidrolysis of the peptide bonds of proteins present in the analyzed mixture; (b) it avoids the cross-linking of peptides of different proteins that possess cystein residues; (c) it facilitates the identification of the proteins in the databases.
***(2) Hydrolisis of the proteins.*** This is achieved with the proteolytic digestion using the lysyl-endopeptidase (LEP) that is highly specific to hydrolize the peptide bonds at the C-terminal end of the lysine residues.
***(3) The blocking reaction*** consists in the chemical covalent modification of the α-amino terminal groups and ε-amino groups of the lysines, present in the peptides generated by the proteolytic treatmentof step 2. In this method a wide variety of modifying amino groups can be used if they guaratees that the resultant modified peptides can be retained selectively in a chromatographic column or in a chemically activated solid support. Among the blocking reagents are: acetic anhydride, N-hydroxysuccinimide, N-acetoxysuccinimide, citacronic anhydride, maleic anhydride, succinic anhydride, ftalic anhydride, tetrahydroftalic anhydride, 9-fluorenylmethyl chloroformate (Fmoc-Cl), 2-methyl sulfonyl ethyl succinimidyl carbonate), urea y reagent that provides protecting amino groups such as: (a) aromatic urethane-type protecting groups which include benzyloxycarbonyl, 2-chlorobenzyloxycarbonyl, 9-fluorenylmethyloxycarbonyl, isonicotinyloxycarbonyl and 4-methoxybenzyloxycarbonyl; (b) aliphatic urethane-type protecting groups which include t-butoxycarbonyl, t-amyloxycarbonyl, isopropyloxycarbonyl, 2-(4-biphenyl)-2-propyloxycarbonyl, allyloxycarbonyl and methylsulfonylethoxycarbonyl; (c) cycloalkyl urethane-type protecting groups which include adamantyloxycarbonyl, cyclopentyloxycarbonyl, cyclohexyloxycarbonyl and isobornyloxycarbonyl; (d) acyl protecting groups or sulfonyl protecting groups. Preferred protecting groups include benzyloxycarbonyl, t-butoxycarbonyl, acetyl, 2-propylpentanoyl, 4-methylpentanoyl, t-butylacetyl, 3-cyclohexylpropionyl, n-butanesulfonyl, benzylsulfonyl, 4-methylbenzenesulfonyl, 2-naphthalenesulfonyl, 3-naphthalenesulfonyl and 1-camphorsulfonyl; (e) photosensitive protective groups which include carbamates derivatives from m-nitrophenyl, 3,5-dimetoxybenzyl, 1-methyl-1(3,5-dimetoxyphenyl)etyl, α-methylnitropiperonyl, o-nitrobenzyl, 3,4-dimetoxy-6-nitrobenzyl, phenyl(o-nitrophenyl)methyl, 2-(2-nitrophenyl)etyl, 6-nitroveratryl, 4-metoxyfenacyl and 3',5'-dimetoxybenzoine and activated esters of the biotin and their chemical derivative. Additionally other blocking groups that provide multiple negative charges to the modified peptide, for example SO₃⁻ groups can also be used. In general it can be used the reagents employed in the peptide synthesis for the protection of amino groups or other reagents able to react with the amino groups that they fulfill the previously explained properties.
   Examples of these kind of reagents and the protocols neccesary to achieve the modification of amino groups can be easily find in the literature (Protective groups in organic synthesis, Teodora W. Greene and Peter G.M. Wuts, pag. 494-654, Ed. John Wiley & Sons, Inc. (1990) y Peptide Chemistry, Bodanszky, N., pag. 74-103, Springer-Verlag, New York (1988) and their usage is comprised in the present invention.
***(4) Destruction* of *the excess of blocking reagent and elimination of the Or-acylation of tyrosine residues.*** This step is carried out with two objectives (1) to destroy the excess of the blocking reagent of the amino groups of LEP peptides and by this way to avoid the reaction with the new amino groups that are generated in the following step during the redigestion with trypsin and (2) to eliminate O-acylations at the tyrosine residues that affect the yield of the proposed method. The addition of an amine such as the ammonia or ethylamine, until achieving approximately a pH 11 and its incubation at 37 °C during one hour guarantees the purposes before mentioned. In particular the destruction of the excess reagent can also be achieved by means of the addition to the reaction buffer of some primary alcohol such as the ethanol or methanol or a mixture of free amino acids.
***(5) Redigestion with trypsin of the LEP peptides blocked at their primary amino groups.*** The blocked LEP peptides are redigested with trypsin, a highly specific enzyme that hydrolize only the peptide bonds at the C-terminal end of arginine in the LEP peptides that contains this amino acid.
***(6) Selective isolation* of *RRnK peptides.*** The used chromatographic column allows the selective isolation of the RRnK peptides with its free amino groups in the non-retained fraction, on the other hand, in the column are retained by means of a covalent or non-covalent linkage the peptides that possess a blocked primary amino group(s) introduced during the step number 3. For the selective isolation of the RRnK peptides any kind of affinity chromatography can be used whenever the blocking group linked to the amino groups is recognized with a very high affinity in this chromatography step. To achieve this, can be used:
   (a) chromatography columns that have immobilized a monoclonal or polyclonal antibody obtained by the hybridome technology; antibodies fragments; single chain antibodies; antibodies isolated from natural source or antibodies selected by means of the phage display libraries.
      Chromatographic columns that has an inmobilized protein or their fragments, or peptides selected by chemical or phage libraries that bind in a highly selective way to the blocking group introduced in the peptides.
   (b) This blocking group can be a natural ligand of the immobilized proteins, a prostetic group or simply a chemical compound of organic or inorganic nature that binds with high specificity to the immobilized protein. For example, the amino group of LEP peptides could be derivatized with biotin or gluthation or some oligosacharidic group in particular to be retained by a column that has immobilized estreptavidin, gluthation S-transferase or a lectin, respectively. Other proteins and their respective ligands can also be used and are summarized by Wang and collaborators (Wang, R.; Fang, X.; Lu, Y.; Wang, S. "The PDBbind Database: Collection of Binding Affinities for Protein-Ligand Complexes with Known Three-Dimensional Structures", J. Med. Chem., 2004; 47(12); 2977-2980 y Wang, R.; Fang, X.; Lu, Y.; Yang, C.-Y.; Wang, S. "The PDBbind Database: Methodologies and updates", J. Med. Chem., 2005; 48(12); 4111-4119.)
   (c) Chromatgraphic column that possesses immobilized metal chelates and that allows the selective retention of those LEP peptides blocked at their amino groups with a histidine tail.

The RRnK peptides can be isolated selectively in the retained fraction when is used a combination of cation exchange chromatography at acidic pH and the derivatization of the primary amino groups of the peptides with a reagent that possesses multiple functional groups that carry several negative charges as they can be for example sulphonic groups (SO₃⁻). All the RRnK peptides at acidic pH possesses two positive charges one located at the N-terminal amino group and the other at the C-terminal arginine, on the contrary, the derivatized peptides with this reagent at acidic pH will have multiple negative charges that will avoids their retention in the cation exchange column. This variant of the method would have the advantage that when being retained the RRnK peptides in the cation exchange chomatography column they could be fractioned additionally previous to its characterization by mass spectrometry to increase the number of identified proteins. Based on the same principle previously described, the anionic exchange chromatography could also be used for retaining all peptides carrying at least a modifying group with negative charges and the RRnK peptides would be isolated selectively in the non-retained fraction.
Also the RRnK peptides can be selected by means of a chemical reaction between the amino blocking groups with a reactive group presents in the chromatographic bead.
(d) for example, if the blocking groups to be used use in the step 3 carry halogens atoms (bromine or iodine fundamentally) located at the primary carbons, or if they possess tioeters or maleimidyl groups or other unsaturated bonds that allow the addition of the sulphidryl groups present in the chromatographic matrices or magnetic beads that have tiols groups in its structure. For these purposes a tiosepharose column can be used (-SH) or a tiolated resins prepared from any resin designed for the peptide synthesis strategy Boc/Bzl (for example the aminomethylated resin , PEG-amino, the Merrifield resin, the 4-methylbencihidrylamine, based on the polyestiren polymer with the necessary modifications) which is coupled a cystein residue. Also it is possible to use as chromatographic column the activated resins with tiols groups like the resin 4-metoxytrityl tiolated, the resin 2-clorotrityl tiolated, the resin N-(2-mercaptoethyl) amine, among others.

The RRnK peptides eluted after the desalting step can be further fractionated using the cation exchange chromatography and reverse phase for the identification of a greater number of proteins (Washburn M. P. et al. Large-scale analysis of the yeast proteome by multidimensional protein identification technology, Nature Biotechnology 19, 242-247, 2001) or to make a double fractionation by reverse phase chromatography to achieve the purpose previously mentioned.
To apply this method in the quantitative proteomics it is necessary that the peptides generated in one of the two compared conditions carry some or several heavy isotopes (¹³C, ¹⁵N, ¹⁸O, and/or ²H) in their structure while the peptides generated in the other condition possesses the elements before mentioned in their natural isotopic abundances (¹²C, ¹⁴N, ¹⁶O, and/or ¹H).
The incorporation of the heavy isotopes in the structure of the peptides generated in one of the two compared conditions can be introduced in two different ways:
***(a) isotopic labeling introduced by the cell.*** It is applied to extracts of proteins obtained from tissues or cells cultivated in media with two isotopic variants of certain essential nutrients for the cell growth. Among the nutrients used are labeled compounds that constitute nitrogen source (¹⁴N/¹⁵N); essential amino acids labeled at certain positions with isotopes of hydrogen (¹H/²H), nitrogen (¹⁴N/¹⁵N), carbon (¹²C/¹³C), oxygen (¹⁶O/¹⁸O), etc. Due to the particularities of this method, all the RRnK peptides possesses at least an arginine residue at their C-terminal end, therefore the introduction of heavy isotopes in this amino acid guarantees the labeling of all the selectively isolated peptides. For example, in one of the compared conditions the culture medium can be enriched in arginine isotopicaly labeled (¹³C₆ - ¹⁵N₂ - or ¹⁵N₄-arginine) and in the other condition the labeling is not carry out. Later on, after mixing both extracts of the total proteins, they are hydrolized at the same time and the method is followed according to that described in the steps 1 to 6 until achieving the selective isolation of the RRnK peptides.
***(b) isotopic labeling introduced during the proteolysis.*** The samples of proteins to be compared are hydrolyzed by separates, as it is described in the step 2 of the method, in aqueous solutions. One of the aqueous solutions was previously prepared with water enriched with ¹⁸O (H₂¹⁸O) and the other one with water that possesses their natural isotopic abundance. By means of this procedure all the proteolytic peptides derived from the first condition are labeled when incorporating one and two atoms of ¹⁸O in their carboxylic C-terminal end. Later on, it proceeds to the inhibition of the proteolytic action of the enzyme used by the addition of a mixture of proteases inhibitors, or specific inhibitors of the proteas used, before mixing equal quantities of the proteins digested in both conditions. It is of highlighting that the labeling of the RRnK peptides particularly takes place in the step 5 during the redigestion of the LEP peptides with trypsin, therefore the addition of reagents before this step should be excluded in order to avid the contamination of the isotopic purity of the H₂¹⁸O used to prepare the digestion solution. When this procedure of isotopic labeling is used, it is not advisable the long treatments of the ¹⁸O-labeled peptides at acidic pH since this can led to the partial or total loss of the labeling introduced enzymatically at the carboxylic C-terminal groups of the peptides and hence introducing big errors in the quantification.
   The relative quantification is carried out from the mass spectra by using appropriate software (Fernández of Cossio et al. Isotopica, A Web Software for Isotopic Distribution Analysis of Biopolymers by Mass Spectrometry. Nuclei Acid Research 2004, 32, W674-W678 and/or Fernández of Cossio et al. Automated Interpretation of Mass Spectra of Complex Mixes by Matching of Isotope Peak Distributions. Rapid Commun. Mass Spectrom. 2004, 18, 2465-2472) to calculate the isotopic distribution of the mixture of labeled and non-labeled RRnK peptides. This software calculates the theoretical isotopic distributions of the labeled and non-labeled RRnK peptides and they are combined in such proportion so that the resultant area of the theoretical isotopic distribution is adjusted from the best way to the area of the isotopic distribution observed experimentally. The existent proportion between each one of the areas corresponding to the labeled peptides with the light and heavy isotopes (¹²C/¹³C, ¹⁴N/¹⁵N, ¹⁶O/¹⁸O, and/or ¹H/²H) once normalized correspond to the relative proportion of the proteins that contain those peptides in the compared mixtures.
   To carry out the quantification it is necessary to know (to) the elementary composition of the analyzed peptide or their sequence, (b) the type of isotopic labeling that has been used in the experiment and (c) the region of the mass spectrum that contains the experimental isotopic distribution of the RRnK peptide of interest. All this information is very restrictive and it allows to calculate with great precision the experimental noise and to discard of the analysis the overlapping of other signals that they are not of interest for the quantification. All these informations make the quantification process with the used software to be very robust and independent of the method of isotopic labeling used.
   This proposed method is compatible with the ionization methods most frequently used in the characterization of peptides and proteins: the electrospray ionization (ESI-MS) and the matrix assisted laser desorption ionization (MALDI-MS). The peptide of interest are selected in the mass spectrometer and they pass through a collision chamber where by using a process known as collision induced dissociation, fragments that contains enough structural information are obtained and allow the deduction of the partial or the complete amino acid sequence of the analyzed peptide. The mass spectrum that contains this information is known as MS/MS spectrum. Each MS/MS spectrum is very typical of the peptide sequence that originated it, and it can be considered as a fingerprint of fragment ions and it is enough for the reliable identification of the peptides in the sequence databases with the help of appropriate softwares. In fact, this it is the principle in which are based the most popular search engines in the identification of the proteins in the sequence databases: the MASCOT software (Matrix Science Ltd, UK) (Perkins, DN, et al. Probability-based protein identification by searching sequence databases using mass spectrometry data. Electrophoresis. 1999, 20, 3551-3567); and the SEQUEST software (Trademark, University of Washington, Seattle Wash. and McCormack, A. L. et al. Direct Analysis and Identification of Proteins in you Mix by LC/MS/MS and Database Searching at the Low-Femtomole Level, Anal. Chem. 1996, 69, 767-776; Eng, J. K. et al. An Approach to Correlate Tandem Mass Spectral Dates of Peptides with Amino Acid Sequences in to Protein Database" J. Am. Soc. Mass. Spectrom. 1994, 5, 976-989; U.S. Pat. No. 5, 538,897 (Jul. 23, 1996) Yates, III et al.).
   These softwares (MASCOT and SEQUEST) compare the MS/MS spectrum obtained experimentally with the theoretical MS/MS spectra of all the peptides that possess a certain molecular mass in the protein sequence databases and have been originated by the specific cleavage of the used protease. The MS/MS spectrum showing the biggest coincidence between the masses values of the theoretical fragments and those obtained experimentally should correspond to that of the analyzed peptide and hence it is inferred the protein that contains it and the identification in the sequence database is performed.
   The following references are related with the application of some techniques of mass spectrometry to the identification of proteins, particularly in the analyzed proteomes: Ideker T, Thorsson V, Ranish J TO, Christmas R, Buhler J, Eng J K, Bungarner R, Goodlett D R, Aebersold R, Hood L. Integrated genomic and proteomic analyses of to systematically perturbed metabolic network. Science. 2001, 292, 929-34; Gygi SP, Aebersold R. Mass spectrometry and proteomics. Curr Opin Chem Biol. 2000, 4, 489-494.; Gygi S P, Rist B, Aebersold R. Measuring gene expression by quantitative proteome analysis. Curr Opin Biotechnol. 2000; 1, 396-401; Goodlett DR, Bruce JE, Anderson GA, Rist B, Passatolic L, Fiehn OR, Smith RD, Aebersold R. Protein identification with a single accurate mass of a cysteine-containing peptide and constrained database searching. Anal. Chem. 2000; 72, 1112-8. and Goodlett DR, Aebersold R, Watts JD. Quantitative in vitro kinase reaction ace to guide for phosphoprotein analysis by mass spectrometry. Rapid Commun Mass Spectrom. 2000; 14, 344-348; Zhou, H. et al. Nature Biotechnol. 2001,19, 375-378.
   Taking into consideration the high selectivity of the proposed method, the identification can be restricted to databases that possess only RRnK peptides to guarantee a faster identification, to avoid of false positive identifications and to obtain a more reliable identification by using MASCOT and SEQUEST softwares.

### FIGURES DESCRIPTION

**FIGURE. 1.** Diagram showing the selective isolation of RRnK peptides using the method described in this invention for its application in the quantitative proteomics. With black rhombuses are indicated the biotin groups added to the primary amino groups of the LEP peptides.

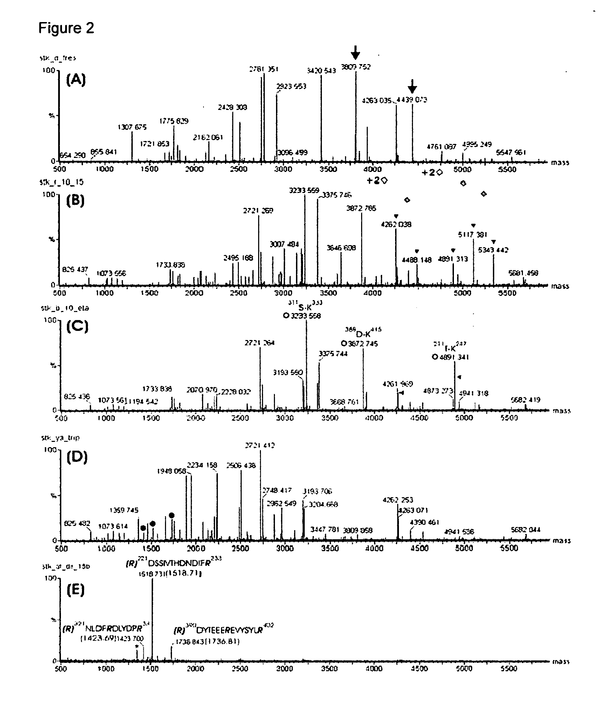

**FIGURE. 2.** The selective isolation of the RRnK peptides is shown in a model protein: the recombinant streptokinase (rSK). (A) ESI-MS spectrum of the mixture of LEP peptides. With black arrows are indicated two LEP peptides that added several blocking groups in the biotinylation reaction (B) ESI-MS spectrum of the peptide mixture after the complete biotinylation of their primary amino groups. With white and black rhombuses are indicated the biotin residues added to the primary amino groups and to the tyrosine residues, respectively. (C) ESI-MS spectrum after the elimination of O-acylation at the tyrosine residues and destruction of the excess of the blocking reagent. With white circles are indicated those blocked LEP peptides that contain the potential RRnK peptides. (D) ESI-MS spectrum of the tryptic digestion of the LEP blocked peptides. With black circles the RRnK peptides of the rSK are indicated. (E) Selective isolation of the RRnK peptides after applying the method object of this invention.
**FIGURE 3.** Chemical reaction between the thiol groups present in a solid support and the blocking groups (maleimide propionyl and iodoacetyl) introduced in the primary amino groups of the LEP peptides. The shady circle indicates the support solid activated with free thiol groups
**FIGURE 4.** The ESI-MS spectra shown in (A) and in (B) correspond to the selective isolation of the RRnK peptides of the rSK using as a solid support the MBHA resin activated with thiol groups and the usage of two blocking groups: maleimidyl and iodoacetyl, respectively.
**FIGURE 5.** ESI-MS spectrum corresponding to the isolation of the RRnK peptides of the rSK using the method proposed in the present invention and the combination of the cation exchange chromatography and the 3,5 disulfo benzoic acid N-hydroxysuccinimide ester as a blocking reagent.

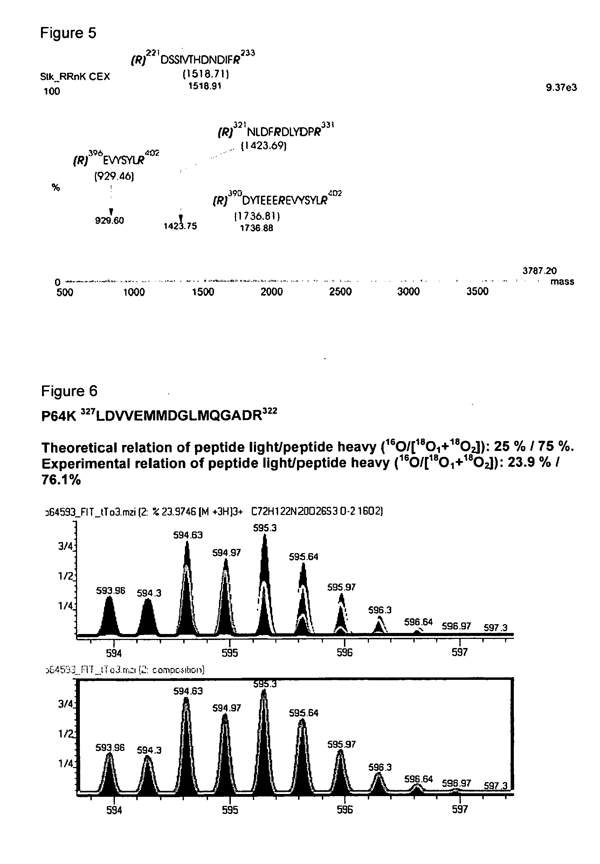

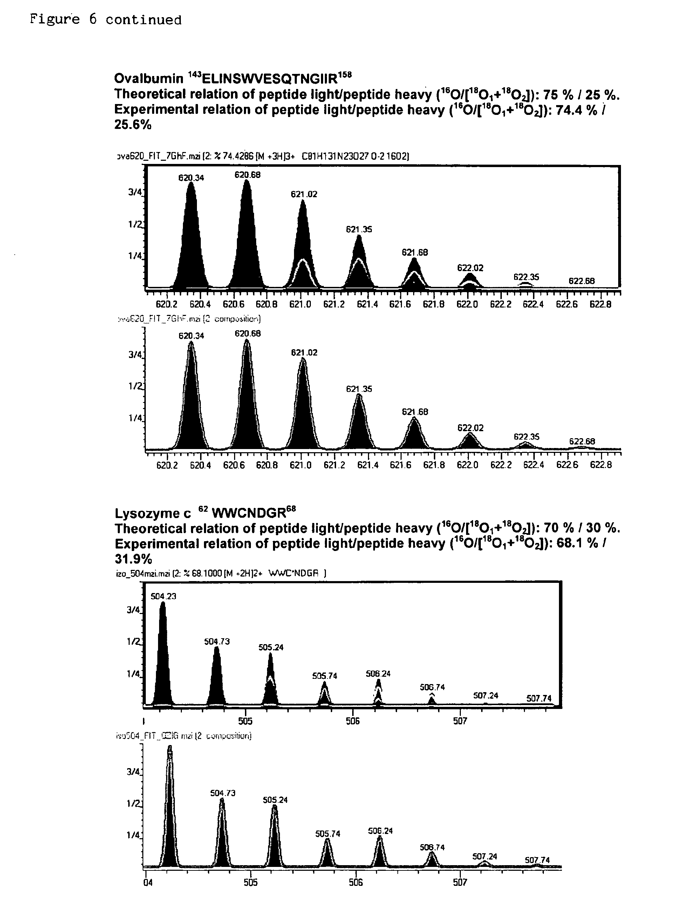

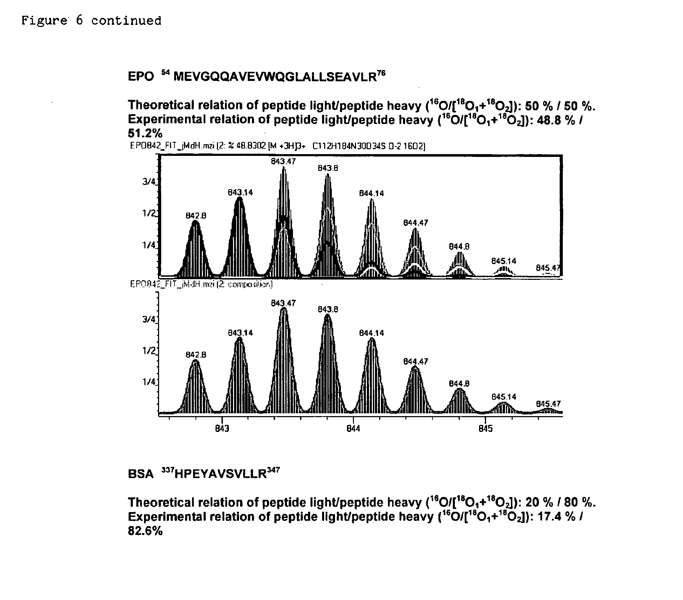

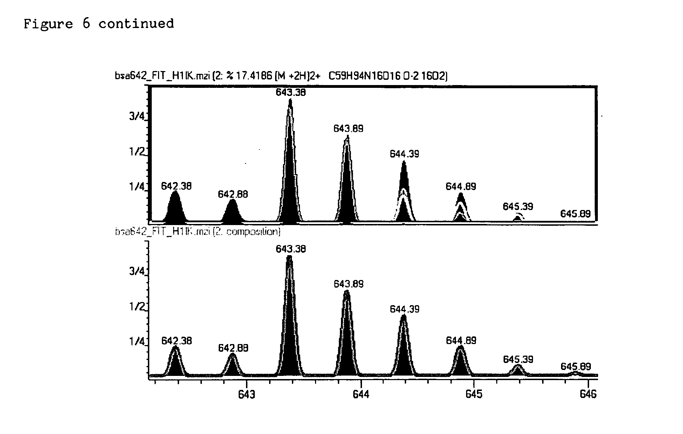

**FIGURE 6.** The ESI-MS spectra shown in black color correspond to the experimental isotopic distributions of the peptides ³²⁷LDVVEMMDGLMQGADR³²² (P64K), ¹⁴³ELINSWVESQTNGIIR¹⁵⁸ (ovalbumin), ⁶²WWCNDGR⁶⁸ (Iysozyme), ⁵⁴MEVGQQAVEVWQGLALLSEAVLR⁷⁶ (EPO), and ³³⁷HPEYAVSVLLR³⁴⁷ (BSA) obtained after applying the method for the selective isolation shown in the example 1, after blocking the groups amino with biotin and using the affinity chromatography with streptavidine immobilized to sepharose and the labeling with ¹⁸O for the relative quantification of the proteins in the two artificial mixtures. In each one of the spectra the isotopic distributions corresponding to the peptides that have two atoms of ¹⁶O₂ (blue color), and one (color violet) and two atoms of ¹⁸O (yellow color) in their C-terminal end are shown. In red color is shown the theoretical isotopic distribution of the mixture of the three species (¹⁶O₂, ¹⁸O₁, and ¹⁸O₂) before mentioned in the proportions shown for each case.

### EXAMPLES.

### Example 1. In silico evaluation of the simplification of complex mixtures of peptides in proteomes of different organisms when applying the method proposed for the selective isolation of RRnK peptides.

Nowadays, the great efficiency reached by the sequencing of the DNA molecule has made possible that full genome sequences of several organisms is known. This in turn allows the prediction of what proteins are derived from these genomes and what peptides will be generated depending on the specific proteolytic treatment that is carried out to the proteome under study.
To appraise which it is the magnitude of the simplification that is possible when isolating selectively the RRnK peptides, a software named SELESTACT was written in C for use in PC that calculates for the proteome of a given organism:
1 - the total number of proteins reported in the Swissprot database,
2 -the total number of peptides/protein that can be generated by a specific proteolytic treatment,
3 -the number of RRnK peptides/protein that are generated by this specific proteolytic treatment,
4 -the proteome coverage that is the percentage of proteins that possess RRnK peptides (referred to the total number of proteins reported for a particular proteome) that can be identified successfully by the method proposed in the present invention.

With comparative purposes this program was also used to calculate these parameters for the proteolytic peptides that possess cystein residues in particular proteome and therefore they could be isolated selectively with success by the ICAT method, one of the pioneering methods and more frequently used in the selective isolation of peptides and its application for proteome studies.

Table 1. *In silico* analysis by using the SELESTACT software of the RRnK peptides contained in the proteome of different organisms when applying the method proposed in Figure 1. The results of the simplification when applying the well-known ICAT method are also shown.

| Analyzed proteome | total number of proteins^{a)} | Total number of tryptic peptides /protein^{b)} | Peptides selectively isolated/protein^{c)} (RRnK / ICAT) | Proteome Coverage^{d)} RRnk/1CAT) |
|---|---|---|---|---|
| *N. meningitidis* | 1967 | 14 | 4/2 | 78.9/81.6 |
| *V. cholerae* | 3785 | 15 | 4/2 | 80.0/80.3 |
| *M. tuberculosis* | 3875 | 15 | 8/2 | 96.3/79.0 |
| *E. coli* | 4322 | 15 | 5/3 | 87.2/83.2 |
| *S. cerevissiae* | 4818 | 24 | 4/4 | 84.3/89.2 |
| *A. thaliana* | 26003 | 14 | 4/5 | 87.7/93.2 |
| *M. musculus* | 28959 | 14 | 5/6 | 88.1/94.7 |
| *H. sapiens* | 47531 | 22 | 6/6 | 88.9/94.8 |
| **Average** | - | 18 | 5/4 | 86.4/87.0 |

| | | | | |
|---|---|---|---|---|
| *a) corresponds to the total number of proteins reported in the Swissprot sequence database.* *b) total number of tryptic peptides that are generated by the specific proteolysis with trypsin of the different analyzed proteomes divided by the total number of coded proteins. It is expressed* as *an integer number.* *c) total number of RRnK peptides per analyzed protein when using the method proposed in figure 1 and the one obtained by the ICAT method. It is expressed* as *an integer number.* *d) the coverage of the analyzed proteome represents the percentage of the total number of proteins that possess RRnK peptides that can be isolated by using the chromatographic method described in the heading of the present Table. The figures shown correspond to the proposed method (left) and to the ICA T method (right).* | | | | |

In the Table 1 the results obtained for several proteomes including pathogens bacteria, yeasts, plants, and mammals are shown. As it can be appreciate, from an average of 18 tryptic peptides/proteins the mixture would be simplified considerably until an optimun value for these purposes because are selectively isolated an average of 5 RRnK peptides/protein which is similar to the one obtained by the ICAT method. The proteome coverage, which represent the percentage of the proteome of an organism that can be studied with the proposed method (86.4%) it is also very similar to the one achieved with the ICAT (87%). However, when individual proteome is analyzed a very remarkable difference is noticed in the case of *M. tuberculosis* proteome. In the method object of the present invention it is possible to analyze 94,6% of the proteome of this organism, on the other hand, when the ICAT is used it can only analyze less than 80%. When studying this microorganism by using proteomic tools the method of choice should be the selective isolation of RRnK peptides object of this invention. The usage of the SELESTACT software is of great utility to predict the results to be expected in particular proteome when applying the method object of the present invention and therefore it allows the comparison with other methods of selective isolation of peptides for proteomics.

This demonstrates us that the principles of this invention, the selective isolation of the RRnK peptides by using a chromatographic system, is of great utility and can be used successfully for the proteomics study of organisms of different evolutionary degree since it allows an ideal simplification of the complex mixture of peptidos and at the same time it guarantees high coverage of the proteome under study.

Only remains to demonstrate in the example 2 an appropriate combination between the blocking of the amino groups and a chromatographic system able to retain in an efficient way the modified peptides while in the non-retained fraction, the RRnK peptides are ready to be analyzed in the mass spectrometer.

### Example 2. Selective isolation of the RRnK peptides of the recombinant streptokinase. Evaluation of the selectivity and the specificity of the method.

The method proposed in the figure 1 was applied to the recombinant streptokinase (rSK, see sequence 1 at the end of the document). This protein was selected as model because its tryptic digestion generates a great quantity of tryptic peptides (42 peptides considering complete cleavage of the used enzyme) and only five RRnK peptides (see Table 2) and it is very easy the evaluation of the specificity and the selectivity of the proposed method.

**Table 2. Amino acid sequence and the theoretical molecular masses of the RRnK peptides of the recombinant streptokinase (rSK).**

| # | Sequence ^{a)} | M+H (theoretical) |
|---|---|---|
| 1 | ***R***↓²²¹DSSIVTHDNDIF***R***²³³↓ | 1518.71 |
| 2 | ***R***↓³²¹NLDF***R***³²⁵↓ | 664.33 |
| 3 | ***R***↓³²⁶DLYDP***R***³³¹↓ | 778.36 |
| 4 | ***R***↓³⁹⁰YTEEE***R***³⁹⁵↓ | 826.35 |
| 5 | ***R***↓³⁹⁶EVYSYL***R***⁴⁰²↓ | 929.46 |

| | | |
|---|---|---|
| *a) The amino acids indicated in boldfaces correspond to the cleavage site of trypsin at the arginine residues. The arrows indicate cleavage site of trypsin.* | | |

The steps to continue were the following ones:
(1) the protein was dissolved in a 500 mM Hepes buffer (pH 8.0) that contains guanidium chloride 2 mol/L, and lysyl-endopeptidase was added using a enzime:substrate ratio of 1:200 and the digestion proceeded at 37 °C during 16 h.
(2) the mixture of proteolytic peptides were incubated at a temperature between 0-5 °C and the modifier reagent (biotinamide pentanoic acid N-hydroxysuccinimide ester) was added in a molar ratio 10:1 respect to the primary amino groups (alpha and epsilon). The mixture was stirred shortly and it was incubated again in a bathroom of ice. This procedure was additionally repeated twice at intervals of five minutes.
(3) the intact modifier reagent and O-acylations at the tyrosine residues were eliminated by incubating the modified LEP peptides to basic pH (2-3 % trietylamine during 1 hour at 37 °C).
(4) the digestion buffer was diluted until obtaining a concentration of 1 mol/L of guanidium chloride and the trypsin was added to an enzyme:substrate ratio of 1:100 and the digestion proceeded additionally during 4h at 37 °C.
(5) the excess of modifying reagent was eliminated by reverse phase chromatgraphy using a RP-C4 column (Vydac, 20 x 2.1 mm) previously equilibrated in a solution of H₂O/TFA 0.05% and the peptides are collected in a single fraction by using a fast gradient when increasing the acetonitrile/TFA (0.05% v/v) content of the mobile phase from 1 to 80% in 10 minutes.
(6) the chromatographic column packed with an affinity matrix (immobilized streptavidine in sepharose) was equilibrated in a 125 mM HEPES buffer (pH 8.0) at a flow of 500 cm/h. The peptide mixture was dissolved in the same equilibrium solution and applied to the affinity column. The absorbance was registered at 226nm and the non-retained fraction was collected for ulterior analysis by mass spectrometry.
(7) the non-retained fraction that contains the RRnK peptides was desalted using the ZipTip (C18) and analyzed in the mass spectrometer.
The ESI-MS spectrum (figure 2A) shows a considerable number of signals corresponding to the peptides generated during the digestion with LEP of the rSK. The assignments of these signals to the sequence of the rSK are shown in the Table 3, as well as the experimental and theoretical mass values of masses for each one of the peptides.

**Table 3. Summary of the assignments of the LEP peptides of rSK to the signals observed in the Figure 2A.**

| # | Assignment^{a)} | m/z exp ^{b)} | m/z theor.^{c)} |
|---|---|---|---|
| 1 | ³⁰⁰YVDVNTNELLK³¹⁰ | 1307.67 | 1307.68 |
| 2 | ³⁸FFEIDLTSRPAHGGK⁵² | 1674.86 | 1674.85 |
| 3 | ²³⁴TILPMDQEFTYHVK²⁴⁷ | 1721.85 | 1721.85 |
| 4 | ³⁷³RPEGENASYHLAYDK³⁸⁷ | 1749.81 | 1749.81 |
| 5 | ²⁵⁹SGLNEEINNTDLISEK²⁷⁴ | 1775.83 | 1775.86 |
| 6 | ²⁸⁰KGEKPYDPFDRSHLK²⁹⁴ | 1816.93 | 1816.93 |
| 7 | ⁶¹SKPFATDSGAMPHKLEK⁷⁷ | 1843.93 | 1843.93 |
| 8 | ²⁵⁸KSGLNEEINNTDLISEK²⁷⁴ | 1903.95 | 1903.95 |
| 9 | ³⁵⁵VEDNHDDTNRIITVYMGK³⁷² | 2120.00 | 2120.00 |
| 10 | ³³⁶LLYNNLDAFGIMDYTLTGK³⁵⁴ | 2162.06 | 2162.08 |
| 11 | ¹⁸⁸TLAIGDTITSQELLAQAQSILNK²¹⁰ | 2428.31 | 2428.32 |
| 12 | ³⁸FFEIDLTSRPAHGGKTEQGLSPK⁶⁰ | 2515.28 | 2515.29 |
| 13 | ³¹¹SEQLLTASER-(***NLDFR***)-(***DLYDPR***)-DK³³³ | 2781.35 | 2781.34 |
| 14 | ¹²³DGSVTLPTQPVQEFLLSGHVRVRPYK¹⁴⁸ | 2923.56 | 2923.57 |
| 15 | ³⁸⁸DR-(***YTEEER***)-(***EVYSYLR***)-YTGTPIPDNPNDK⁴¹⁵ | 3420.54 | 3420.59 |
| 16 | ⁸³AIQEQLIANVHSNDDYFEVIDFASDATITDRNGK¹¹⁶ | 3809.76 | 3809.82 |
| 17 | ³⁵⁵VEDNHDDTNRIITVYMGKRPEGENASYHLAYDK³⁸⁷ | 3850.76 | 3850.80 |
| 18 | ¹MIAGPEWLLDRPSVNNSQLWSVAGTVEGTNQDISLK³⁷ | 3938.00 | 3938.03 |
| 18 | ³³⁶LLYNNLDAFGIMDYTLTGKVEDNHDDTNRIITVYMGK³⁷² | 4263.01 | 4263.06 |
| 20 | ²¹¹THPGYTIYER-(***DSSIVTHDNDIFR***)TILPMDQEFTYHVK²⁴⁷ | 4439.08 | 4439.13 |

| | | | |
|---|---|---|---|
| *a) amino acids highlighted in boldfaces and italic correspond to the sequences of the five RRnK peptides of the rSK. The numbering of the N - and C-terminal amino acids of each peptide correspond to the position of each one in the sequence of the rSK.* *b) experimental mass of the LEP peptides of the rSK.* *c) theoretical mass of the LEP peptides of the rSK.* | | | |

Notice in Table 3 that LEP peptides 13, 15 and 20 contain the sequences of five RRnK peptides of the rSK sequence shown in the Table 2.
Most of the LEP peptides increased their molecular masses in a quantity corresponding to the number of blocking groups added at their primary amino groups (amino terminal and amino epsilon groups of the lysines) contained in their structures. Most of the LEP peptides originated by a complete cleavage should add two blocking groups (2 x 226 Da =552 Da) one at the free amino N-terminal group and the other at the lysine residue located at the C-terminal end. However, some of these peptides added a quantity of blocking groups higher than the expected. For example, in the figure 2B it can be clearly appreciated how the signals of masses 3809.75 and 4439.07 that correspond to the biotinylated peptides 16 and 20 of the Table 3, added one and two additional blocking groups than the expected (see number of added shady rhombuses, in figure 2B).
A detailed analysis of the ESI-MS/MS corresponded to these modified peptides demonstrated that these additions are located at sidechain of the tyrosine reside. These results agreed with the obtained by Zappacosta and Annan (Zappacosta F, and Annan RS. N-terminal isotope tagging strategy for quantitative proteomics: results-driven analysis of protein abundance changes. Anal Chem. 2004, 76, 6618-6627) when they tried to block in a quantitative way all the amino terminal groups of the tryptic peptides with another reagent.
Later on, this undesirable modification at the tyrosine residues was eliminated with a basic treatment (Figures 2C) mentioned in the step 3 of the described in the present example (Zappacosta F, and Annan RS. N-terminal isotope tagging strategy for quantitative proteomics: results-driven analysis of protein abundance changes. Anal Chem. 2004, 76, 6618-6627). Notices, that the signals that possess dark rhombuses in the figure 2B after basic treatment are absent in the figure 2C therefore it demonstrate that the basic treatment is effective to revert the effect of this side reaction. The signals indicated with white circles in figure 2C correspond to biotinylated LEP peptides that possess RRnK peptides within their sequence. To generate the RRnK peptides, the biotinylated LEP peptides are digested with trypsin. The resulting mixture is shown in the figure 2D. The signals labeled with black circles in the figure 2D correspond to the RRnK peptides of the rSK.
To achieve the selective isolation of the RRnK peptides, the reaction mixture passed through a sepharose column with inmobilized streptavidine and the non-retained fraction was analyzed by mass spectrometry and the results are shown in the figure 2E. Three signals of masses 1423.70, 1518.71 and 1736.84, were assigned to the RRnK peptides, NLDFRDLYDPR, DSSIVTHDNDIFR and YTEEEREVYSYLR respectively. Of these three RRnK peptides, only one of them is contained in Table 2 (see peptide 1). The other two RRnK peptides of masses 1423.70 and 1736.84 isolated selectively in the figure 2E were originated by an uncomplete cleavage of trypsin and they contain the remaining four RRnK peptides shown in Table 2. These uncomplete cleavages of trypsin could be due to the presence of acidic residues (Asp) contiguous to the arginine residues.
The signal at mass 1343.66 labeled with an asterisk in figure 2E do not correspond with a non-specific isolation of a non-RRnK peptide and it is assigned to a fragment (series y"₁₁) of the RRnK peptide ³⁹⁰YTEEEREVYSYLR⁴⁰² of mass 1736.80 originated in the ionization source during the mass spectrometric measurement.
These results demonstrate that the proposed method can be used with success in proteomic experiments because it simplified considerably the complex mixture of peptides shown in the ESI-MS spectrum of the figure 2D and it was able to isolate selectively without any unespecificity the five RRnK peptides (shown in Table 2) theoretically predicted for the model protein rSK.
On the other hand, the high selectivity and specificity of the method can restrict the search in the databases by the software devised for these ends, to only RRnK peptides to avoid in the false-positive identifications.
One of the disadvantages that possesses the ICAT method, one of the most used methods in proteomics for the selective isolation of peptides without using the two-dimensional electrophoresis, is the high molecular mass of the reagent introduced in all the cystein-containing peptides because it can affect their ionization efficiency and fragmentation in the ulterior mass spectrometric analysis.
On the contrary, the RRnK peptides does not have in their structure any type of chemical modification introduced during the steps of the method and it does not affect the ionization and fragmentation in gas phase during the collision induced dissociation experiments. This advantage is very appreciated to obtain an efficient fragmentation of the peptides in gas phase and to guarantee a more reliable identification in the sequence databases.

### Example 3. Selective isolation of the RRnK peptides using chemically active solid support.

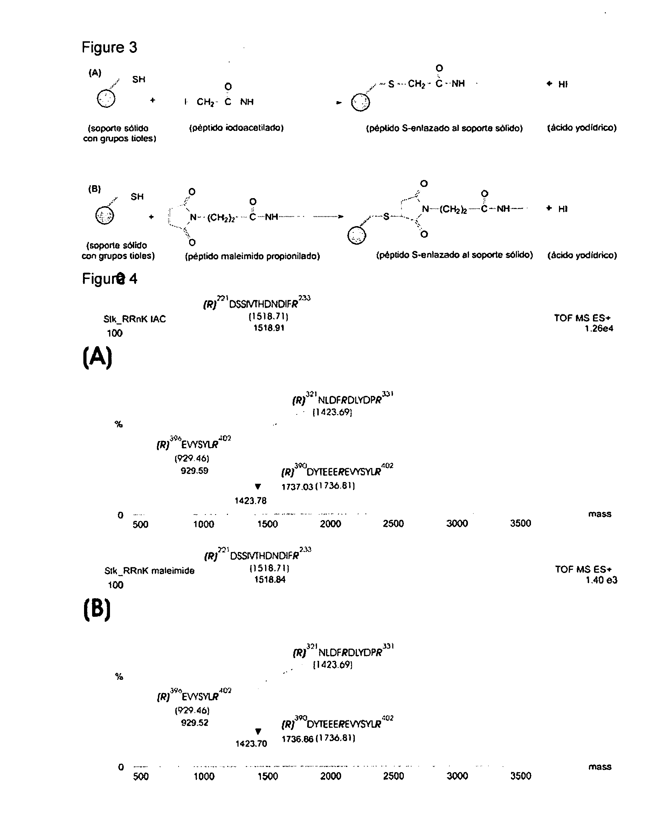

The RRnK peptides can be isolated by using solid supports funcionalized with chemically active groups by a reaction with the modifier group introduced during the blocking reaction of the amino groups of the LEP peptides. By this way a covalente bound is formed between the modified peptides and the solid support previuously described. On the other hand, since the RRnK peptides do not have any chemical modification in its structure they do not react with the solid support and are isolated selectively in the non-retained fraction and are analyzed by mass spectrometry.
In the present example, is shown the selective isolation of RRnK peptides by using a solid support (MBHA methyl benzidrylamine resin) that contains free thiols groups and the chemical modification of the LEP peptides with two different chemical reagents (iodoacetic acid N-hydroxysuccinimide ester and maleimide propionic acid N-hydroxysuccinimide ester) that react in a quantitative way with the thiols groups as shown in the figure 3.
The preparation of the solid support was carried out in the following way:
Four excesses of the mixture of Fmoc-L-Cys(Trt)-OH/HOBt/DIC (1/1/1) dissolved in dimethylformamide were added to the MBHA resin (with a substitution of 1-1.2 mmol/g) previously activated. The reaction continued until the Kaiser assay is negative. The Fmoc-L-Cys(Trt) is treated with a mixture of 20 % piperidine in dimethylformamide during 20 minutes to eliminate the group Fmoc that protects the amino terminal end. Later on, it is washed several times with DMF. The Cys(Trt) resin is washed several times with methanol and finally with ether. It is kept in vaccum during 24 h and to eliminate the Trt groups that protect the tiols groups of Cys it react with the mixture of TFA/EDT/water/TIS (94/2.5/2.5/1) during 2 h. Finally the resin is filtered and washes thoroughly with ether.
The steps to follow for isolating the RRnK peptides of the rSK protein were identical to the steps (1) to the (4) described in the example 3 of the present invention, with the difference that in the step (2) the blocking of the amino groups was carried out separately with two different reagents the iodoacetic acid N-hydoxysuccinimide ester and the maleimide propionic acid N-hydoxysuccinimide ester. The following steps are described below:
(5) the solid support, previously hydrated in the same solution where the rSK was dissolved, was added to the reaction mixture maintaining a molar ratio of 50:1 of the thiols groups respect to the quantity of the added reagent. The reaction mixture was kept at room temperature, in the darkness, during 4h with low stirring and the iodoacetic acid N-hydoxysuccinimide ester and the maleimide propionic acid N-hydoxysuccinimide ester were used, respectively.
(6) the matrix linked to the modified peptides was eliminated by centrifugation to 10 000 rpm during five minutes and washed twice with the same reaction buffer.
(7) the supernatant that contains the RRnK peptides of the rSK was desalted by using the ZipTips and analyzed by mass spectrometry.

The results obtained when finishing the steps of the previously described procedure were very similar to each other and it can be appreciated in the ESI-MS spectra obtained when the two reagents were used: the iodoacetic acid N-hydoxysuccinimide ester (figures 4A) and the maleimide propionic acid N-hydoxysuccinimide ester (figures 4B). It can be appreciated that in both cases (figures 4A and B) the signals at m/z 929.59, 1423.78, 1518.91 and 1737.03 are the most intense in the obtained ESI-MS spectrum and these masses values are in good agreement with the expected ones for the RRnK peptidos of the rSK protein (see results of the example 2, Figures 2E and Table 2).

Beside the above mentioned signals no others signals of appreciable intensity were detected therefore it does not suggests the non-specific isolation of non-RRnK peptides in the non-retained fraction. These results demonstrated us that the usage of a chemical reaction between the solid support and the blocking group introduced at the primary amino groups of the LEP peptides is useful for the selective isolation of the RRnK peptides for proteomics studies. The usage of these matrices has the advantage that when having a high concentration of activated functional groups on its surface, a small volume of the matrix can be used and by this way guarantee not only the quantitative linkage of the modified peptides but also the linkage of excess of reagent added to achieve a quantitative blocking of the amino groups of the LEP peptides. Particularly this simplifies the method of selective isolation of the RRnK peptides by eliminating a desalting step (see step 5 of the example 2) before the selective isolation.
This example, also demonstrates the concept that using quantitative chemical reactions between the modifying reagents of primary amino groups introduced during the blocking step of LEP peptides and the functional group of a solid matrix can be used for the highly selective and specific isolation of the RRnK peptides.
On the other hand, although in this example the solid support linked to the modified peptides was eliminated by a simple centrifugation process, however this could be achieved by magnetic field when magnetic beads as solid support are used whenever they are appropriately activated to capture the modified peptides by the formation of a covalent linkage with the modifying group at the primary amino groups.

### Example 4. Usage of the cation exchange chromatography for the selective isolation of the RRnK peptides.

In the examples 2 and 3, the RRnK peptides are isolated in the non-retained fraction by using the affinity chromatography and the usage of a chemical reaction between an activated solid support and the blocking group introduced in the amino groups of the LEP peptides, respectively. In the present example, it is demonstrated that the cation exchange chromatography combined with the derivatization of the amino groups with reagents that introduce negative charges to the peptides can also be used for this purpose. This idea is based in the principle that RRnK peptides do not carry any modification in its terminal amino group and possess an arginine at their C-terminal end, and once they are dissolved at acidic pH should possess, at least, two positive charges, and if they possess some histidine residues within their sequences the number of positive charges would be increased. It means that RRnK peptides are peptides carrying multiple positive charges.
On the other hand, the remaining peptides originated by the tandem digestion LEP/trypsin when being blocked their primary groups amino, either their amino N-terminal end or the side chains of the lysine residues, a protonation site is substracted and therefore the possibility to acquire a positive charge is eliminated. If additionally, the blocking group of amino groups possesses negative charges, the net negative charge of the peptide will increase considerably.
In the following Table is shown the charges that should acquire the RRnK peptides and the modified peptides of the rSK after derivitizing their amino groups with a reagent that additionally contributes with two negative charges even at acidic pH because it is originated from an strong acid. For example, a modifier reagent that fulfills these characteristics can introduce sulphonic groups (SO₃⁻) to the peptides.

**Table. 4. Analysis of the relative quantity of the positive and negative charges of the proteolytic peptides obtained in the proposed method once their amino groups were derivatized with the 3,5-disulfobenzoic acid N-hydroxysuccinimide ester.**

| # ^{a)} | Amino acidic Sequence ^{b)} | # SO₃^{-d)} | # (R+H+Nₜ) ^{e)} | Z ^{f)} |
|---|---|---|---|---|
| 1 | ¹MIAGPEWLLD**R**PSVNNSQLVVSVAGTVEGTNQDISL*K*³⁷ | 4 | 1 | 3- |
| 2 | ³⁸FFEIDLTS**R**PA**H**GG*K*⁵² | 4 | 2 | 2- |
| 3 | ⁵³TEQGLSP*K*⁶⁰ | 4 | 0 | 4- |
| 4 | ⁶¹S*K*PFATDSGAMP**H***K*⁷⁴ | 6 | 1 | 5- |
| 5 | ⁷⁵LE*K*⁷⁷ | 4 | 0 | 4- |
| 6 | ⁷⁸ADLL*K*⁸² | 4 | 0 | 4- |
| 7 | ⁸³AIQEQLIANV**H**SNDDYFEVIDFASDATITD**R**¹¹³ | 2 | 2 | 0 |
| 8 | ¹¹⁴NG*K*^{116 b)} | 2 | 1 | 1- |
| 9 | ¹¹⁷VFAD*K*¹²² | 4 | 0 | 4- |
| 10 | ¹²³DGSVTLPTQPVQEFLLSG**H**V**R**¹⁴³ | 2 | 2 | 0 |
| 11 | ¹⁴⁴V**R**PY*K*^{148 c)} | 2 | 2 | 0 |
| 12 | ¹⁴⁹E*K*PIQNQA*K*¹⁵⁷ | 6 | 0 | 6- |
| 13 | ¹⁵⁸SVDVEYTVQFTPLNPDDDF**R**PGL*K*¹⁸¹ | 4 | 1 | 3- |
| 14 | ¹⁸²D**T***K*¹⁸⁴ | 4 | 0 | 4- |
| 15 | ¹⁸⁵LL*K*¹⁸⁷ | 4 | 0 | 4- |
| 16 | ¹⁸⁸TLAIGDTITSQELLAQAQSILN*K*²¹⁰ | 4 | 0 | 4- |
| 17 | ²¹¹T**H**PGYTIYE**R**²²⁰ | 2 | 2 | 0 |
| 18 | ²²¹SSIVT**H**DNDIF**R**^{233 c}) | 0 | 3 | 3+ |
| 19 | ²³⁴TILPMDQEFTY**H**V*K*²⁴⁷ | 2 | 2 | 0 |
| 20 | ²⁴⁸N**R**²⁴⁹ | 2 | 2 | 0 |
| 21 | ²⁵⁰EQAYEIN*K*^{257 c)} | 2 | 1 | 1- |
| 22 | ²⁵⁹SGLNEEINNTDLISE*K*²⁷⁴ | 4 | 0 | 4- |
| 23 | ²⁷⁵YYVL*K*²⁷⁹ | 4 | 0 | 4- |
| 24 | ²⁸¹GE*K*PYDPFD**R**²⁹⁰ | 4 | 1 | 3- |
| 25 | ²⁹¹S**H**L*K*^{294 c)} | 2 | 2 | 0 |
| 26 | ²⁹⁵LFTI*K*²⁹⁹ | 4 | 0 | 4- |
| 27 | ³⁰⁰YVDVNTNELL*K*³¹⁰ | 4 | 0 | 4- |
| 28 | ³¹¹SEQLLTASE**R**³²⁰ | 2 | 1 | -1 |
| 29 | ³²¹NLDF**R**^{325c)} | 0 | 2 | 2+ |
| 30 | ³²⁶DLYDP**R**^{331c)} | 0 | 2 | 2+ |
| 31 | ³³²D*K*^{333b)} | 2 | 1 | 1- |
| 32 | ³³⁶LLYNNLDAFGIMDYTLTG*K*³⁵⁴ | 4 | 0 | 4- |
| 33 | ³⁵⁵VEDN**H**DDTN**R**³⁶⁴ | 2 | 2 | 0 |
| 34 | ³⁶⁵IITVYMG*K*^{372c)} | 2 | 2 | 0 |
| 35 | ³⁷⁴**R**PEGENASY**H**LAYD*K*³⁸⁷ | 4 | 2 | 2- |
| 36 | ³⁸⁸D**R**³⁸⁹ | 2 | 1 | 1- |
| 37 | ³⁹⁰YTEEE**R**^{395c)} | 0 | 2 | 2+ |
| 38 | ³⁹⁶EVYSYL**R**^{402c)} | 0 | 2 | 2+ |
| 39 | ⁴⁰³YTGTPIPDNPND*K*^{415c)} | 2 | 0 | 2- |

| | | | | |
|---|---|---|---|---|
| *a) The shadow rows contain the RRnK peptides of the rSK.* *b) the amino acids highlighted in boldfaces correspond to the basic amino acids histidine and arginine. The lysine residues modified with the sulfonic groups are highlighted in italic. The shown peptides were generated by the complete cleavage of LEP and trypsin.* *c) Peptides generated by the cleavage of the trypsin that contain their free amino terminal end.* *d) Quantity of negative charges introduced by the presence of sulfonic group in the sequence of the modified peptides.* *e) positive charges provided by the basic amino acids arginine and histidine and the amino terminal end of peptides generated by the cleavage with trypsin.* *f) Sum of the negative and positive charges of each one of the peptides.* | | | | |

The net charge of the peptide at acidic pH was considered as the sum of the positive charges (apported by the presence of basic residues of arginine and histidine and the amino terminal group in those peptides that possess free amino terminal group (R+H+Nt)) and the negative charges provided by the two sulfonic groups present in the modifier reagent added to the primary amino groups of the LEP peptides.
The results show that the five RRnK peptides of the rSK bear multiple positive charges (z=2+ to 3+), on the other hand, the remaining peptides can be neutral or even have negative charges. This demonstrates that the separation of these pools of peptides by cation exchange chromatography is feasible. To introduce two negative charges for each modified amino group the 3,5-disulfobenzoic acid N-hydroxysuccinimide ester was synthesized using the following protocol:
1 mol of 3,5-disulfobenzoic acid dissolved in THF react with 1 mol of diisopropyl carbodiimide and 1.5 moles of NHS during 20 hours. The precipitate is filtered and the supernatant is rotoevaporated. The resultant precipitate is crystallizes in ethyl acetate.
To isolate the RRnK peptides using cation exchange chromatopgraphy the same steps were followed (1)-(5) as described in the example 2 with the difference that in the step 2 the 3,5-disulfobenzoic acid N-hydroxysuccinimide ester dissolved in the same reaction buffer was added to the reaction mixture (HEPES 500 mM, pH 8.0) to block the primary amino groups of the LEP peptides.
The next steps are described below:
(6) the cation exchange chromatographic column (10 x 5 mm) was packed with the EMD-650 (S) SO₃⁻ matrix from Merck company and it was equilibrated in a solution of TFA (0.05% v/v). The peptides, dissolved in the same equilibrium buffer but containing additionally octylglucoside 0.5% (w/v), were applied to the column at a flow of 500 cm/h. The absorbance was registered at 226nm. The non-retained fraction was discarded; the column was washed extensively to eliminate the remains of the detergent used. The retained peptides were eluted by applying the same equilibrium solution that contains 2 mol/L NaCl. The retained fraction was collected for its analysis by mass spectrometry.
(8) the non-retained fraction that contains the RRnK peptides was desalted using the ZipTip (C18) and analyzed in the masses spectrometer.
The chromatography system described here, has been used with success for the separation of positively-charged peptides from the neutral peptides in methods devised for the selective isolation of the peptides that contains the C-terminal end of proteins (Isolation and characterization of modified species of to mutated (Cys¹²⁵-Ala) recombinant human interleukin-2. Moya G, González LJ, Huerta V, García Y, Mulberry V, Pérez D, Heath F, M. Claws J Chromatogr A. 2002, 971(1-2), 129-42.) and the blocked N-terminal end (Selective isolation and identification of N-terminal blocked peptides from tryptic protein digests. Betancourt L, Besada V, González LJ, Takao T, Shimonishi Y. J. Pept. Res. 2001, 57(5), 345-53) of the proteins and more recently their use has been extended to the selective isolation of peptides for proteomic studies (SCAPE: A new tool for the Selective Captures of Peptides in Protein identification. Betancourt L, Gil J, Besada V, González LJ, Fernández-de-Cossío J, García L, Pajón R, Sánchez A, Alvarez F, Padrón G. J. Proteome Res. 2005, 4, 491-496).
When the results shown in Table 4 are analyzed it can be clearly appreciated that five RRnK peptides of rSK (peptides 18, 29, 30 37 and 38) are the only ones that possess net positive charges at acidic pH once their amino groups have been modified with a reagent that introduce two sulfonic groups. On the contrary, all the remaining peptides since they are carrying this modification their net charge is negative or neutral therefore it is expected that this chromatography system designed to separate the positively-charged peptides from the neutral ones can also be used successfully for the selective isolation of the RRnK peptides in the retained fraction.
This was demonstrated when obtaining the ESI-MS spectrum of the retained fraction where only all the expected RRnK peptides of rSK were detected (Figure 5). Although the signals at masses 1423.75 and 1736.88 are not included in the Table 4 of the present example are important to point out that they correspond to the peptides ³²¹N-R³³¹ and ³⁹⁰D-R⁴⁰² and they are also classified as RRnK because they are originated by an specfic cleavage at the arginine residues and they do not contain lysine residues within their sequences. These peptides are originated by the incomplete cleavage of the trypsin and they contain the sequences of the RRnK peptides (29 and 30) and (37 and 38) shown in the Table 4.
Equally, the anionic exchange chromatography could be used for the selective isolation of the RRnK peptides because the modified peptides have multiply-negative charges and they can be easily retained, on the contrary the RRnK peptides is the only ones that do not have sulfonic groups in their structure and they would be isolated in the non-retained fraction. In this case, the RRnK peptides would be isolated in the non-retained fraction.
It is necessary to highlight that while more negative charges are carried by the modified peptides, the selectivity and specificity of the proposed method should be higher and then an easier separation of the modified and the RRnK peptides by using the cation exchange chromatography can be achieved.
Once the amino groups of the LEP peptides are blocked they are transformed in more hydrophobic species and in some cases their solubility can it turns be compromised, however when modifying with charged groups they should guaranteed an increment of their solubility in aqueous media with respect to the reagents used in the previous examples. This should contribute to diminish the losses associated to the blocking of the amino groups proposed in the method of the present invention.
The same chromatografic column used for the selective isolation of the RRnK peptides can be used for the additional fractionation of the peptides of interest. This not only it simplifies the method but also by alternating saline gradients and of reverse phase in a similar way as it is carried out in the MudPiT experiments (Washburn M. P. et al. Large-scale analysis of the yeast proteome by multidimensional protein identification technology, Nature Biotechnology 19, 242-247, 2001) the identification of a bigger number of proteins can be achieved.
The results obtained in the present example demonstrate that the cation exchange chromatography in combination with an appropriate derivatization of the amino groups of peptides that introduces negative charges can be used with success for the selective isolation of the RRnK peptides in proteomic experiments.

### Example 5. Identification and relative quantification of the component proteins in two artificial mixtures (A and B) by the selective isolation of RRnK peptides using the isotopic labeling with ¹⁸O.

Two artificial mixtures A and B, composed each one for the proteins rSK, recombinant human eritropoietin (EPO), lysozyme C, ovalbumin, P64K and BSA was prepared. The molar ratio A/B was different for each protein: 1:1 for the rSK, 2:1 for the lysozyme, 3:1 for the ovalbumin, 1:3 for the P64K and 1:5 for the BSA. The sequences of these proteins are shown in the listing of sequences (1-6) of the present document.
The mixtures of proteins A and B at a concentration of 5 mg/ml were dissolved separately in a 200mM HEPES buffer (pH 8.0) containing 2 mol/L guanidium chloride and 10 mM EDTA. A 50 fold molar excess of DTT respect to the concentration of cystein residues was added and the reaction mixture was incubated in nitrogen atmosphere at 37 °C during 4 hours. The solution was cooled down to room temperature and twice molar excess of acrylamide solution respect to the quantity of DTT previously added and was kept in the darkness for a period of 1 hour. Both mixtures were digested with the LEP protease during 8 hours at 37 °C using an enzyme:substrate ratio of 1:100. The reaction mixture cooled down to a temperature 0-5°C and three additions of the blocking reagent (biotinamide pentanoic acid N-hydroxysuccinimide ester) to intervals of 20 minutes were carried out using a proportion 8:1 with respect to the concentration of primary amino groups of the generated peptides. Later on, triethylamine is added to a concentration of 5% and the reaction is incubated at 37 °C during 1 hour. The resulting mixtures are liophylized, and reconstituted independently using normal water (mixture A) and ¹⁸O-labeled water (H₂¹⁸O) of 99% of isotopic purity provided by ISOTEC (mixture B). Both are digested with trypsin using a proportion 1:20 at 37 °C. After 4 hours, an aprotinine solution at a concentration 2:1 respect to the quantity of trypsin was added to both mixtures to stop the proteolysis and to avoid the isotopic exchange catalyzed by the enzyme once the generated labeled and non-labeled peptides were mixed. Both mixtures of peptides are mixed and desalted by reverse phase chromatography using a fast gradient of acetonitrile to eliminate the excess of the added blocking reagent. The mixture of peptides is concentrated and dissolved in the same equilibrium solution (HEPES, 200mM, pH 8.0) of the affinity matrix composed by streptavidine immobilized in sepharose. The sample was applied to a flow of 2 cm/h and the non-retained fraction was desalted and analyzed by LC-MS/MS.
During the proteolysis of the mixture B, the peptides labeled with ¹⁸O can add 1 and 2 atoms of ¹⁸O at their C-terminal end and it is necessary to keep in mind to calculate the relative quantities of the peptides obtained under both conditions because the ratio is given by the ratio of the areas of the isotopic distributions corresponding to the peptides that have ¹⁶O₂ divided by the sum of the areas of the distributions of the peptides that incorporated one (¹⁸O₁) and two atoms of 18-oxygen (¹⁸O₂), according to the expression: (Area ¹⁶O₂)/(Area ¹⁸O₁+ Area ¹⁸O₂).
The relative quantification of the peptides in the analyzed mixtures is carried out by using the ISOTOPICA software as it is explained in the detailed description of the present invention method (Fernández of Cossio et al. Isotopica, A Web Software for Isotopic Distribution Analysis of Biopolymers by Mass Spectrometry. Nuclei Acid Research 2004, 32, W674-W678 and/or Fernández of Cossio et al. Automated Interpretation of Mass Spectra of Complex Mixtures by Matching of Isotope Peak Distributions. Rapid Commun. Mass Spectrom. 2004; 18: 2465-2472).
The RRnK peptides of the six proteins present in the prepared mixtures were isolated and sequenced in a single LC-MS/MS experiment and the results of the identification carry out by the MASCOT software as well as the results of the quantification appear in the Table 3.
Particularly, to proceed to the quantification, the expanded region of the isotopic distribution of these peptides was selected and introduced in the ISOTOPICA software together with the global formula of the analyzed peptide and the labeling with ¹⁸O.

**Table 3. Summary of the selective isolation of the RRnK peptides and their relative quantification of five proteins present in two artificial mixtures labeled with ¹⁸O/¹⁶O.**

| Proteins | Sequence of RRnK peptides ^{a)} | Quantification | |
|---|---|---|---|
| | | Theor. ^{b)} | Exp. ^{c)} |
| EPO | ⁵⁴MEVGQQAVEVWQGLALLSEAVLR⁷⁶ | 50:50 | 48.1:51.9 |
| | ¹⁰⁴SLTTLLR¹¹⁰ | | 49.1:50.9 |
| | ¹³⁴TITADTFR¹³⁹ | | 52.2:47.8 |
| | ¹⁴⁴VYSNFLR¹⁵⁰ | | 52.4:47.6 |
| | | | **50.6:49.4 (± 2.2)** |
| Lysozyme C | ¹⁵HGLDNYR²¹ | 66:33 | 67.1:32.9 |
| | ⁴⁶NTDGSTDYGVLQINSR⁶¹ | | 66.9:33.1 |
| | ⁶²WWCNDGR⁶⁸ | | 68.1:31.9 |
| | | | **67.4:32.6 (± 0.6)** |
| Ovalbumin | ¹²⁷GGLEPINFQTAADQAR¹⁴² | 75:25 | 76.2:23.8 |
| | ¹⁴³ELINSWVESQTNGIIR¹⁵⁸ | | 74.3:25.7 |
| | | | **75.3:24.8** (± 1.3) |
| P64K | ³²²LDVVEMMDGLMQGADR³³⁷ | 25:75 | 23.2:76.8 |
| | ³⁸⁶YDAVLVAAGR³⁹⁵ | | 24.6:75.4 |
| | | | **23.9:76.1** (± 1.0) |
| BSA | ³³⁷HPEYAVSVLLR³⁴⁷ | 15:85 | 16.1:83,9 |
| | ⁴⁴⁵MPBTEDYLSLILNR⁴⁵⁸ | | 17.0:83.0 |
| | | | **16.6:83.4 (± 0.6)** |

| | | | |
|---|---|---|---|
| *a) sequence of the RRnK peptides* of *the proteins identified automatically by the MASCOT software.* *b) theoretical ratio of the proteins in the compared artificial mixtures A and B.* *c) experimental values obtained when determining the relative quantities of the proteins present in the two compared artificial mixtures. In boldfaces letters is indicated the average of their relative quantification and the value of the standard deviation between parenthesis.* | | | |

For the six proteins the average of the experimental values corresponding to the relative quantifications are in excellent agreement with the theoretical ones and the obtained standard deviation was very small (below 5%).
These results demonstrate that the method can be used in the quantitative proteomics to determine with very good accuracy the relative quantities of the proteins present in mixtures. The adjustments of the areas corresponding to the experimental isotopic distributions obtained for one peptide of the identified proteins are shown figure 3.
Notice you that in all the cases very good adjustment was obtained between the theoretical contour of the theoretical distributions (red line) and the spectrum obtained experimentally (spectrum shadowed in black). The results obtained for the relative quantification of the peptides belonging to each one of the proteins were very similar to the theoretical values which demonstrate the utility of the method in quantitative proteome studies.
The method for the selective isolation of RRnK peptides is completely compatible with the ¹⁸O-labeling and it allows its application to the quantitative proteomic study of any biological system because the steps comprised in the isolation method do not affect the labeling introduced at the C-terminal end of the peptides. The labeling method ¹⁶O/¹⁸O can be introduced in an universally in all the peptides generated during the proteolysis of the compared protein mixtures.
The coincidence between the experimental and theoretical values for the relative quantifications of each one of the proteins confirms us that the ISOTOPICA software offers reliable results even when the used isotopic labeling (¹⁸O) does not assure the separation of the isotopic distributions of the labeled and non-labeled peptides.
Since in this method of selective isolation all the RRnK peptides possesses arginine at their C-terminal end, it is predictable that the usage of the methodology known as SILAC that introduces the specific labeling with ¹³C₆ at the arginine residues (Gruhler A, Schulze WX, Matthiesen R, Mann M, Jensen ON. Stable isotope labeling of Arabidopsis thaliana cells and quantitative proteomics by mass spectrometry. Mol Cell Proteomics. 2005, 4(11),1697-709) could be used for the determination of the relative quantification. In a same way, any other type of labeling introduced during the culture conditions (¹H/²H or ¹⁴N/¹⁵N) could be used at the arginine residues.

### LC-MS/MS and database search.

The measurements were carried out in a hybrid mass spectrometer (quadrupole and time of flight, QTof-2) from the Micromass company (Manchester, United Kingdom). The mass spectrometer was connected online with a HPLC (AKTA Basic, Amersham Pharmacia Biotech, Sweden) through a column of 200 x 1 mm (Vydac, USES). The peptides were eluted with a lineal gradient from 5 to 45 % of the buffer B (0.2% of formic acid in acetonitrile) in 120 minutes.
The mass spectrometer was operated with cone and the capillary voltages of 35 and 3000 volts, respectively. For the acquisition of the MS/MS spectra the singly-, doubly-, triply-charged precursory ions were selected automatically, once these they surpassed an intensity of 7 counts/seg. The measurement mode was changed from MS/MS to MS when the total ion current (TIC) diminished to 2 counts/seg or when the spectra MS/MS was acquired during 4 seconds. The acquisition and the data processing were carried out by the MassLynx software (version 3.5, Micromass, UK), while the identification of the proteins based on the spectra MS/MS was through the version of the MASCOT software freely available in Internet. Among the search parameters, the cystein modification as well as the possible oxidations and desamidations were included.

## Claims

1. - Method for the identification and relative quantification of one or several proteins in complex mixtures originated from a cellular extract or a biological fluid, **characterized by** the selective isolation of peptides originated by the cleavage at the C-terminal end of arginine residues (RR) and do not contain lysine residues within their sequences (nK), denominated here as RRnK peptides, where the determination of the relative concentrations of the proteins is carried out by ratio between the areas corresponding to the theoretical spectra for each RRnK peptide labeled with different isotopes in the compared samples, which consists of the following steps:
**a)** desnaturalization and S-alkylation of the cystein residues of the proteins present in the analyzed complex mixture, and specific hydrolisis of their peptide bonds at the C-terminal end of the lysine residues using the enzyme lysyl endopeptidase (LEP).
**b)** reversible or irreversible chemical modification of the primary groups amino (alpha and epsilon) of the peptides obtained in the step (a) with a such reagent that in the step (e) it can be retained in a chromatographic column or in an activated solid support by non-covalent interactions (electrostatic, affinity, hydrophobicity, etc) or by the formation of covalent bonds.
**c)** alkaline treatment for the destruction of the excess of added blocking reagent, and the elimination of those O-acylations at the tyrosine residues.
**d)** digestion of the LEP blocked peptides obtained in the step (b) with trypsin.
**e)** differential isotopic labeling of the samples of proteins during the cultivation conditions using the methodology known as SILAC previous to the step (a) or labeling of the peptides during the steps (a) and/or (d).
**f)** retention of the peptides that carry the blocking groups introduced during the step (b) in the chromatographic column or in an activated solid support by means of any kind of interaction, covalent or non-covalent with high affinity, and obtaining the RRnK peptides in the non-retained or retained fraction depending the case.
**g)** identification of the proteins that contain the RRnK peptides isolated selectively in the step (e) by mass spectrometry coupled to the liquid chromatography.
**h)** relative quantification of one or several proteins in the mixtures of the step (g) from the ratio between the areas of the estimated theoretical mass spectra for the RRnK peptides identified in the step (f).

2. - The method of the claim 1, step (b), **characterized by** the covalent modification of the α-amino terminal y ε-amino de las lysine contained in the peptides generated during the proteolytic treatment, step (a), using modifying reagents of amino groups such as: acetic anhydride, N-hydroxysuccinimide, N-acetoxysuccinimide, citacronic anhydride, maleic anhydride, succinic anhydride, ftalic anhydride, tetrahydroftalic anhydride, 9-fluorenylmethyl chloroformate (Fmoc-Cl), 2-methyl sulfonyl ethyl succinimidyl carbonate), urea y reagent that provides protecting amino groups such as: (a) aromatic urethane-type protecting groups which include benzyloxycarbonyl, 2-chlorobenzyloxycarbonyl, 9-fluorenylmethyloxycarbonyl, isonicotinyloxycarbonyl and 4-methoxybenzyloxycarbonyl; (b) aliphatic urethane-type protecting groups which include t-butoxycarbonyl, t-amyloxycarbonyl, isopropyloxycarbonyl, 2-(4-biphenyl)-2-propyloxycarbonyl, allyloxycarbonyl and methylsulfonylethoxycarbonyl; (c) cycloalkyl urethane-type protecting groups which include adamantyloxycarbonyl, cyclopentyloxycarbonyl, cyclohexyloxycarbonyl and isobornyloxycarbonyl; (d) acyl protecting groups or sulfonyl protecting groups. Preferred protecting groups include benzyloxycarbonyl, t-butoxycarbonyl, acetyl, 2-propylpentanoyl, 4-methylpentanoyl, t-butylacetyl, 3-cyclohexylpropionyl, n-butanesulfonyl, benzylsulfonyl, 4-methylbenzenesulfonyl, 2-naphthalenesulfonyl, 3-naphthalenesulfonyl and 1-camphorsulfonyl; (e) photosensitive protective groups which include carbamates derivatives from m-nitrophenyl, 3,5-dimetoxybenzyl, 1-methyl-1 (3,5-dimetoxyphenyl)etyl, 0-methylnitropiperonyl, o-nitrobenzyl, 3,4-dimetoxy-6-nitrobenzyl, phenyl(o-nitrophenyl)methyl, 2-(2-nitrophenyl)etyl, 6-nitroveratryl, 4-metoxyfenacyl and 3',5'-dimetoxybenzoine and activated esters of the biotin and their chemical derivative. Additionally other blocking groups that provide multiple negative charges to the modified peptide, for example SO₃⁻ groups. In general it can be used the reagents employed in the peptide synthesis for the protection of amino groups or other reagents able to react with the amino groups that they fulfill the previously explained properties.

3. - The method of the claim 1, step (a), **characterized by** the usage of any type of affinity matrices that have immobilized any monoclonal antibody or antibody fragments obtained by phage display libraries, or single chain antibodies, peptides obtained by synthetic procedures or isolated from natural sources or isolated from synthetic peptide libraries or by phage display peptide libraries. Other proteins with high affinity for binding to a natural or artifitial ligands introduced at the amino groups of the LEP peptides and in a general way activated matrices containing reactive groups that can bind in a covalent or non-covalent way the blocking group introduced in the amino groups of peptides.

4. - The method of the claim 1, **characterized by** the usage of cation exchange chromatography in combination with the derivatization of the amino groups to incorporate negative charges, for isolating selectively in the retained fraction the RRnK peptides.

5. - The method in agreement with the claim 1, step (g), **characterized by** the determination of the relative concentration of one or several proteins in the samples by the calculating the ratios of the areas corresponding to the theoretical spectra of the labeled and non-labeled the species of the RRnK peptides which are adjusted in the best way to the mass spectra obtained experimentally of the peptides generated in the step (e).
